(19) Europäisches Patentamt
European Patent Office
Office européen des brevets

(11) Veröffentlichungsnummer: **0 004 624**
**B1**

(12) **EUROPÄISCHE PATENTSCHRIFT**

(45) Veröffentlichungstag der Patentschrift: **24.02.82**

(21) Anmeldenummer: **79100905.3**

(22) Anmeldetag: **24.03.79**

(51) Int. Cl.³: **C 07 D 311/22,**
C 07 D 311/96,
C 07 D 311/92,
C 07 D 401/10,
A 61 K 31/35, A 23 K 1/00

(54) **Neue Chromanon-Derivate, Verfahren zu ihrer Herstellung und ihre Verwendung als Arzneimittel und Wachstumsförderer.**

(30) Priorität: **07.04.78 DE 2814983**

(43) Veröffentlichungstag der Anmeldung:
**17.10.79 Patentblatt 79/21**

(45) Bekanntmachung des Hinweises auf die Patenterteilung:
**24.02.82 Patentblatt 82/8**

(84) Benannte Vertragsstaaten:
**BE CH DE FR GB IT NL SE**

(56) Entgegenhaltungen:
**DE - A - 2 535 338**

(73) Patentinhaber: **BAYER AG**
**Zentralbereich Patente, Marken und Lizenzen**
**D-5090 Leverkusen 1, Bayerwerk (DE)**

(72) Erfinder: **Widdig, Arno, Dr.**
**Eifgenstrasse 8**
**D-5068 Odenthal (DE)**
Erfinder: **Kabbe, Hans-Joachim, Dr.**
**Walter-Flex-Strasse 16**
**D-5090 Leverkusen 1 (DE)**
Erfinder: **Scheer, Martin, Dr.**
**Claudiusweg 9**
**D-5600 Wuppertal 1 (DE)**
Erfinder: **Sitt, Rüdiger, Dr.**
**Claudiusweg 9**
**D-5600 Wuppertal 1 (DE)**

**EP 0 004 624 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue Chromanon-Derivate, Verfahren zu ihrer Herstellung und ihre Verwendung als Arzneimittel und Wachstumsförderer

Durch die Erfindung werden neue Chromanon-Derivate der Formel I

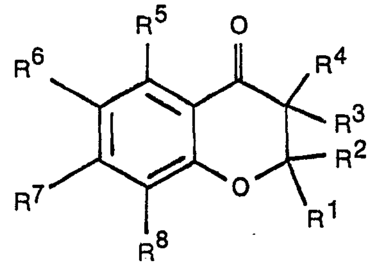

(I)

bereitgestellt, in welcher

$R^1$ und $R^2$ gleich oder verschieden sind und für Wasserstoff, einen geradkettigen oder verzweigten Alkylrest mit bis zu 18 C-Atomen, einen geradkettigen oder verzweigten Alkylenrest mit bis zu 18 C-Atomen, einen $C_3$—$C_{18}$-Cycloalkylrest, einen $C_3$—$C_{18}$-Cycloalkenylrest, einen $C_6$—$C_{10}$-Arylrest, einen $C_7$—$C_{18}$-Aralkylrest mit 1 bis 8 Kohlenstoffatomen im aliphatischen und 6 bis 10 Kohlenstoffatomen im carbocyclischen Teil, einen $C_2$—$C_7$-Alkoxycarbonylrest, einen Dialkylaminoalkylrest mit 1 bis 6 Kohlenstoffatomen im Alkylteil oder für einen 5 bis 7-gliedrigen Heterocyclenrest mit 1 bis 3 gleichen oder verschiedenen Heteroatomen aus der Reihe Sauerstoff, Schwefel oder Stickstoff stehen oder zusammen mit dem durch sie eingeschlossenen C-Atom einen gesättigten oder ungesättigten carbocyclischen, gegebenenfalls an einen oder mehrere Reste aus der Benzolreihe anellierten 3- bis 12-gliedrigen Ring bilden oder zusammen mit dem durch sie eingeschlossenen C-Atom einen, gegebenenfalls ein oder mehrere Heteroatome aus der Reihe Sauerstoff, Schwefel oder Stickstoff enthaltenden 5- bis 12-gliedrigen, gegebenenfalls eine oder zwei Doppelbindungen enthaltenden heterocyclischen Ring bilden,

$R^5$, $R^6$, $R^7$ und $R^8$ gleich oder verschieden sind und für Wasserstoff, Hydroxy, Nitro, Cyano, Carboxy, Halogen, einen geradkettigen oder verzweigten Alkylrest mit bis zu 18 C-Atomen, einen $C_6$—$C_{10}$-Arylrest, einen $C_7$—$C_{18}$-Aralkylrest mit 1 bis 8 Kohlenstoffatomen im aliphatischen und 6 bis 10 Kohlenstoffatomen im carbocyclischen Teil, einen $C_1$—$C_6$-Alkoxyrest, einen $C_2$—$C_7$-Alkoxycarbonylrest, einen $C_6$—$C_{10}$-Aryloxyrest, einen $C_7$—$C_{10}$-Aralkoxyrest, einen Alkylamino-, Alkenylamino-, Arylamino-, Aralkylamino- oder Dialkylaminorest mit bis zu 8 Kohlenstoffatomen oder für Fluor, Chlor, Brom oder Jod stehen, $R^3$ für den Substituenten

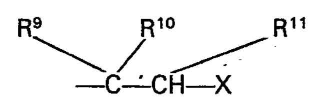

steht, wobei $R^9$, $R^{10}$ und $R^{11}$ gleich oder verschieden sind und für Wasserstoff, Cyano oder Nitro oder für einen geradkettigen oder verzweigten Alkylrest mit bis zu 18 C-Atomen, $C_3$—$C_{18}$-Cycloalkylrest, $C_6$—$C_{10}$-Arylrest, $C_7$—$C_{18}$-Aralkylrest mit 1 bis 8 Kohlenstoffatomen im aliphatischen und 6 bis 10 Kohlenstoffatomen im carbocyclischen Teil, $C_1$—$C_6$-Alkoxyrest, $C_2$—$C_7$-Alkoxycarbonylrest oder $C_1$—$C_6$-Acylrest stehen und X für Cyano, Nitro oder für die Reste $COR^{12}$, $SO_2R^{12}$ oder $PO(R^{12})_2$ steht, in denen $R^{12}$ Wasserstoff, Hydroxy, die Aminogruppe, Methylamino-, Äthylamino-, Propylamino-, Isopropylamino-, Dimethylamino-, Diäthylamino, Dipropylamino-, Diisopropylamino-, Phenyläthylamino-, Anilino-, Benzylamino-, Piperidino-, Pyrrolidino-, N-Phenyl-N-methylamino- oder Morpholinogruppe, einen geradkettigen oder verzweigten Alkylrest mit bis zu 18 C-Atomen, $C_6$—$C_{10}$-Arylrest, $C_7$—$C_{18}$-Aralkylrest mit 1 bis 8 Kohlenstoffatomen im aliphatischen und 6 bis 10 Kohlenstoffatomen im carbocyclischen Teil, $C_1$—$C_6$-Alkoxyrest, $C_6$—$C_{10}$-Aryloxyrest, $C_7$—$C_{10}$-Aralkoxyrest, $C_2$—$C_6$-Alkenyloxyrest oder 5 bis 7-gliedrigen Heterocyclenrest mit 1 bis 3 gleichen oder verschiedenen Heteroatomen aus der Reihe Sauerstoff, Schwefel oder Stickstoff bedeutet, oder wobei $R^9$ und $R^{10}$, $R^{10}$ und $R^{11}$, $R^9$ und $R^{12}$ oder $R^{11}$ und $R^{12}$ zusammen mit den durch sie eingeschlossenen Kohlstoffatomen einen gesättigten oder ungesättigten carbocyclischen, gegebenenfalls an einen oder mehrere Reste aus der Benzolreihe anellierten, 3- bis 12-gliedrigen Ring bilden,

$R^4$ für Wasserstoff, $R^1$ oder $R^3$ steht und in welcher die Reste $R^1$ bis $R^{12}$ in der Bedeutung Alkyl, Cycloalkyl, Cycloalkenyl, Aryl, Aralkyl, Alkoxy und Alkoxycarbonyl durch Halogen, den Cyanorest, $C_1$—$C_6$-Alkyl, $C_1$—$C_6$-Alkoxy, $—NH_2$, $C_1$—$C_6$-Alkylamino sowie durch Alkoxycarbonyl oder Dialkylamino mit jeweils 1—6 C-Atomen in den Alkyl- bzw. Alkoxyresten substituiert sein können.

Besonders bevorzugt sind Verbindungen der Formel I in welcher $R^1$ und $R^2$ die in Anspruch 1 angegebene Bedeutungen bebesitzen,

$R^3$ für den Substituenten

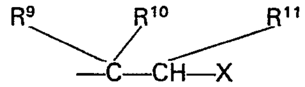

steht, wobei $R^9$, $R^{10}$ und $R^{11}$ gleich oder verschieden sind und für Wasserstoff oder $C_1$—$C_4$-Alkyl stehen,

$R^4$ für Wasserstoff, $R^1$ oder $R^3$ steht,

$R^5$, $R^6$, $R^7$ und $R^8$ gleich oder verschieden sind und für Wasserstoff, Halogen, $C_1$—$C_4$-Alkyl, Hydroxy, $C_1$—$C_4$-Alkoxy, $C_6$—$C_{10}$-Aryloxy, $C_7$—$C_{10}$-Aralkoxy stehen,

X für die Cyanogruppe oder die Reste $COR^{12}$ oder $SO_2R^{12}$ steht, wobei $R^{12}$ für $C_1$—$C_{12}$-Alkyl steht.

Die Chromanone der Formel I werden zweckmäßigerweise durch Umsetzung von Chromanonen der Formel II

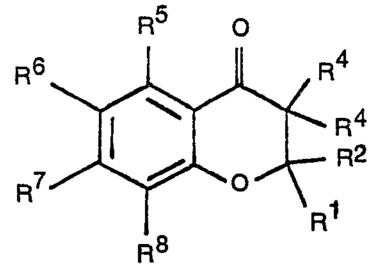

(II)

in welcher $R^1$, $R^2$, $R^4$, $R^5$, $R^6$, $R^7$ und $R^8$ die oben angegebene Bedeutung besitzen, mit Olefinen der Formel III

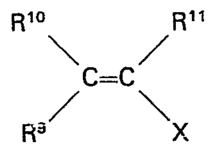

(III)

in welcher $R^9$, $R^{10}$, $R^{11}$ und X die oben angegebene Bedeutung haben, in Gegenwart basischer Hilfsstoffe hergestellt.

Überraschenderweise zeigen die erfindungsgemäßen Chromanonderivate eine erheblich höhere hypocholesterinämische Wirksamkeit als das aus dem Stand der Technik bekannte Clofibrat. Außerdem zeigen sie eine überraschend stark ausgeprägte nutritive Wirkung, welche bei Verbindungen dieser Stoffklasse bislang noch nicht gefunden wurde.

Chromanone-(4) werden in der DE—OS 2 611 910 sowie in Elderfield, Heterocylic Compounds, Vol. 2, Seite 347 und in Bull. Soc. Chem. Belge *82*, 705 (1973) beschrieben. Über ihre Wirksamkeit als Nutritiva werden hier jedoch keine Aussage gemacht.

Verwendet man 2-Spirocyclopenta-chromanon-(4) und Methylvinylketon als Ausgangsprodukte, so kann der Reaktionsablauf durch das folgende Formelschema wiedergegeben werden:

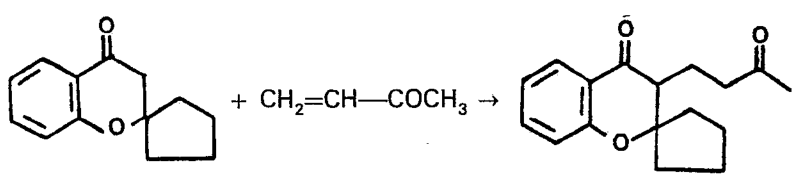


In den Formeln I, II bzw. III stehen als gegebenenfalls substituiertes Alkyl $R^1$, $R^2$, $R^4$, $R^5$, $R^6$, $R^7$, $R^8$, $R^9$, $R^{10}$, $R^{11}$ und $R^{12}$: Methyl, Äthyl, Propyl, Isopropyl, Butyl, Isobutyl, tert.-Butyl, Hexyl, 2-Hexyl-, 1,1-Dimethylpentyl, 1,1-Dimethylhexyl, 1,1-Dimethylheptyl, Nonyl, Decyl, Undecyl oder Octadecyl.

Als Alkenylreste $R^1$, $R^2$ stehen bevorzugt Äthenyl, Propenyl-(1), Propenyl-(2), Butenyl-(3), 4-Methylpentenyl-(3), 4,8-Dimethylnondienyl-(3,7), 4,8,12-Trimethyltridecatrienyl (3,7,11).

Als gegebenenfalls substituierte Cycloalkylreste $R^1$, $R^2$, $R^9$, $R^{10}$ und $R^{11}$ kommen vorzugsweise solche mit 4 bis 12, insbesondere bevorzugt mit 5 und 6 Kohlenstoffatomen, wie Cyclobutyl, Cyclopentyl und Cyclohexyl in Frage.

Als gegebenenfalls substituierte Cycloalkenylreste $R^1$ und $R^2$ kommen vorzugsweise solche mit 4 bis 12, insbesondere bevorzugt mit 5 und 6 Kohlenstoffatomen, vorzugsweise 5 und 6-gliedrige Alicyclen mit einer Doppelbindung wie Cyclopentenyl-(1), Cyclopentenyl-(2), Cyclopentenyl-(3), Cyclohexenyl-(1), Cyclohexenyl-(2), Cyclohexenyl-(3), 4-Methylcyclohexen-(3)-yl in Frage.

Als gegebenenfalls substituiertes Aryl $R^1$, $R^2$, $R^4$, $R^9$, $R^{10}$, $R^{11}$, $R^{12}$, $R^5$, $R^6$, $R^7$ und $R^8$ seien beispielhaft gegebenenfalls substituiertes Phenyl oder Naphthyl genannt.

Als gegebenenfalls substituierte Aralkylreste $R^1$, $R^2$, $R^4$, $R^9$, $R^{10}$, $R^{11}$, $R^{12}$, $R^5$, $R^6$, $R^7$ und $R^8$ kommen

vorzugsweise solche, deren aliphatischer Teil 1 bis 4 Kohlenstoffe enthält und deren aromatischer Teil einen carbocyclischen Rest mit 6 bis 10 Kohlenstoffatomen darstellt, in Frage. Beispielsweise seien die folgenden Aralkylreste genannt: Benzyl, Phenyläthyl, Phenylpropyl, Phenylbutyl, Naphthylmethyl und Naphthyläthyl, bevorzugt Benzyl.

Als gegebenenfalls substituiertes Alkoxy $R^9$, $R^{10}$, $R^{11}$, $R^{12}$, $R^5$, $R^6$, $R^7$ und $R^8$ steht gerädkettiges oder verzweigtes Alkoxy mit vorzugsweise 1 bis 4 Kohlenstoffatomen. Beispielhaft seien gegebenenfalls substituiertes Methoxy, Äthoxy, n.- und i.-Propoxy und n.-, i.- und t.-Butoxy genannt.

Als gegebenenfalls substituiertes Alkoxycarbonyl $R^1$, $R^2$, $R^9$, $R^{10}$, $R^{11}$, $R^5$, $R^6$, $R^7$ und $R^8$ steht geradkettiges oder verzweigtes Alkoxycarbonyl mit vorzugsweise 2 bis 5 Kohlenstoffatomen. Beispielhaft seien gegebenenfalls substituiertes Carbomethoxy, Carboäthoxy, Carbo-n.- und i.-Propyloxy und Carbo-n.-, i.- und t.-Butyloxy genannt.

Als bevorzugte Aryloxygruppen $R^{12}$, $R^5$, $R^6$, $R^7$ und $R^8$ seien Phenoxy und Naphthoxy genannt.

Als bevorzugte Aralkoxygruppen $R^{12}$, $R^5$, $R^6$ $R^7$ und $R^8$ seien Benzyloxy, Phenyläthoxy, Phenylpropoxy, Phenylisopropoxy, Phenylbutoxy, Phenylisobutoxy und Phenyl-tert.-butoxy genannt.

Als Halogene $R^5$, $R^6$, $R^7$ und $R^8$ seien Fluor, Chlor, Brom und Jod, bevorzugt Brom und Chlor, genannt.

Als Acyl $R^9$, $R^{10}$, $R^{11}$ steht Acyl mit vorzugsweise 2 bis 4 Kohlenstoffatomen. Beispielhaft seien genannt: Acetyl, n-Propionyl, i-Propionyl, n-Butyryl, i-Butyryl. Außerdem steht Acyl vorzugsweise auch für Cycloalkylcarbonyl oder Arylcarbonyl wie z.B. Cyclohecylcarbonyl oder Benzoyl.

Als Dialkylaminoalkyl $R^1$, $R^2$ steht Dialkylaminoalkyl mit vorzugsweise 1 bis 4 Kohlenstoffatomen im jeweiligen Alkylteil. Beispielhaft seien genannt: N,N Dimethylaminomethyl, N,N-Diäthylaminomethyl, N,N-Dimethylaminoäthyl, N,N-Diäthylaminoäthyl, N,N-Methyläthylaminomethyl.

Als Alkenyloxyreste $R^{12}$ stehen Alkenyloxyreste mit vorzugsweise 2 bis 4 Kohlenstoffatomen im Alkenylteil, beispielhaft seien auufgeführt: Vinyloxy, Allyloxy, Methallyloxy.

Als gegebenenfalls substituierte Heterocyclenreste $R^1$, $R^2$, $R^{12}$ stehen heteroparafinische, heteroaromatische und heteroolefinische vorzugsweise 5- oder 6-gliedrige Ringe mit vorzugsweise 1 oder 2 gleichen oder verschiedenen Heteroatomen. Als Heteroatome stehen Sauerstoff, Schwefel oder Stickstoff. Als Beispiele seien gegebenenfalls substituiertes Pyrrolidinyl, Piperidinyl, Furyl, Thienyl, Pyrazolyl, Imidazolyl, 1,2,3- und 1,2,4-Triazolyl, Oxazolyl, Isoxazolyl, Thiazolyl, Isothiazolyl, 1,2,3-, 1,3,4-, 1,2,4- und 1,2,5-Oxadiazolyl, Azepinyl, Pyrrolyl, Isopyrrolyl, Pyridyl, Piperazinyl, Pyridazinyl, Pyrimidinyl, Pyrazinyl, 1,3,5-, 1,2,4- und 1,2,3-Triazinyl, Oxepinyl, Morpholinyl und Thiepinyl genannt.

Falls die jeweiligen Substituentenpaare $R^1$ und $R^2$ zusammen mit dem durch sie eingeschlossenen Kohlenstoffatom, $R^9$ und $R^{10}$ zusammen mit dem durch sie eingeschlossenen Kohlenstoffatom, $R^{10}$ und $R^{11}$ zusammen mit den durch sie eingeschlossenen Kohlenstoffatomen, $R^5$ und $R^6$ zusammen mit den durch sie eingeschlossenen Kohlenstoffatomen, $R^7$ und $R^8$ zusammen mit den durch sie eingeschlossenen Kohlenstoffatomen einen carbocyclischen Ring bilden, falls $R^9$ und $R^{12}$ zusammen mit den durch sie eingeschlossenen Kohlenstoffatomen einen carbocyclischen Ring bilden oder falls $R^{11}$ und $R^{12}$ zusammen mit den durch sie eingeschlossenen Kohlenstoffatomen einen carbocyclischen Ring bilden, so kommen gesättigte oder ungesättigte Kohlenwasserstoffglieder enthaltende Ringe, bevorzugt 3- bis 12-gliedrige Ringe wie Cyclopropan, Cyclopentan, Cyclohexan, Cycloheptan, Cyclooctan, Cyclononan, Cyclodecan, Cycloundecan, Cyclododecan, Cyclohexen, Cycloocten, Cyclododecen und Tetralin in Frage. Es ist auch möglich, daß die carbocyclischen Ringe an einem oder mehrere Reste aus der Benzolreihe anelliert sind.

Falls die jeweiligen Substituentenpaare $R^1$ und $R^2$ zusammen mit dem durch sie eingeschlossenen Kohlenstoffatom einen gegebenenfalls substituierten heterocyclischen Ring bilden, so kommen vorzugsweise 5- bis 12-gliedrige Ringe, insbesondere 5- und 6-gliedrige Ringe wie Piperidin, N-Methyl- und N-Benzylpiperidin, Pyrollidin, Tetrahydrofuran, Tetrahydropyran und Tetrahydrothiopyran in Frage. Die heterocyclischen Ringe können an einen oder mehrere Reste aus der Benzolreihe anelliert sein.

Als Substituenten der Alkyl-, Cycloalkyl-, Cycloalkenyl-, Aryl-, Aralkyl-, Alkoxy-, Alkoxarbonyl-Reste der Substituenten $R^1$, $R^2$, $R^4$, $R^9$, $R^{10}$, $R^{11}$, $R^{12}$, $R^5$, $R^6$, $R^7$ und $R^8$ kommen Substituenten in Frage, die unter den Reaktionsbedingungen nicht verändert werden. Beispielsweise seien die Halogene, wie Fluor, Chlor, Brom und Jod, die Cyangruppe, $C_1$—$C_6$-Alkylgruppe, die $C_1$—$C_6$-Alkoxygruppe, die $C_1$—$C_6$-Alkoxycarbonylgruppe, die Aminogruppe, die $C_1$—$C_6$-Alkylamino- und die $C_1$—$C_6$-Dialkylaminogruppe, Arylreste aus der Benzolreihe oder die Carboxylgruppe genannt.

Die bei der Herstellung der erfindungsgemäßen Verbindungen eingesetzten Chromanone-(4) der Formel II sind zum großen Teil bekannt (vgl. hierzu in der Reihe Heterocyclic Compounds "Chromenes, Chromanones and Chromones", 207 ff, Ed. G. P. Ellis, New York, London, Sydney, Toronto 1977 und die DE—OS 2 611 910). Sie können im übrigen nach analogen Verfahren hergestellt werden.

Als mögliche Herstellungsverfahren für Chromanone-(4) seien beispielhaft aufgeführt:

a) Kondensation von o-Hydroxyacetophenon mit Carbonylverbindungen in Gegenwart basischer Kondensationsmittel.

b) Fries-Umlagerung der Phenolester $\alpha,\beta$-ungesättigter Carbonsäuren.

c) Cyclisierung von $\beta$-Phenoxypropionsäuren mit sauren Kondensationsmitteln.

d) Reduktion von Chromonen.

Als Beispiele für Ausgangschromanone der Formel II seien genannt:

Chromanon-(4), 2-Methyl-chromanon-(4), 6-Chlor-chromanon-(4),
7-Chlor-chromanon-(4), 6,8-Dichlor-chromanon-(4),
6-Methyl-chromanon-(4), 7-Methyl-chromanon-(4),
6,7-Dimethyl-chromanon-(4), 6,8-Dimethyl-chromanon-(4),
6-Methoxy-chromanon-(4), 7-Methoxy-chromanon-(4),
6-Aethoxy-chromanon-(4), 7-Aethoxy-chromanon-(4),
6-Chlor-2-methyl-chromanon-(4), 7-Chlor-2-methyl-chromanon-(4),
2,6-Dimethyl-chromanon-(4), 2,6,7-Trimethyl-chromanon-(4),
6-Methoxy-2-methyl-chromanon-(4),
7-Methoxy-2-methyl-chromanon-(4),
6-Chlor-2-aethyl-chromanon-(4),
7-Chlor-2-aethyl-chromanon-(4),
6-Methyl-2-aethyl-chromanon-(4), 2-Aethyl-chromanon-(4),
2-Propyl-chromanon-(4), 2-Isopropyl-chromanon-(4),
2-Butyl-chromanon-(4), 2-sec.Butyl-chromanon-(4),
2-tert.-Butyl-chromanon-(4), 2,2-Diaethyl-chromanon-(4),
2,2-Dimethyl-chromanon-(4), 2-Methyl-2-propyl-chromanon-(4),
2-n-Hexyl-chromanon, 2-Aethyl-7-methoxy-chromanon-(4),
7-Methoxy-2-propyl-chromanon-(4),
2-Isopropyl-7-methoxy-chromanon-(4),
2-Butyl-7-methoxy-chromanon-(4),
2-sec.Butyl-7-methoxy-chromanon-(4),
2-tert.-Butyl-7-methoxy-chromanon-(4),
2,2-Diaethyl-7-methoxy-chromanon-(4),
2,2-Dimethyl-7-methoxy-chromanon-(4),
2-Spirocyclopenta-chromanon-(4),
2-Spirocyclohexa-chromanon-(4),
2-Phenyl-chromanon-(4), 2-Cyclohexyl-chromanon-(4),
2-Benzyl-chromanon-(4), 2-Phenylaethyl-chromanon-(4),
2-Phenylpropyl-chromanon-(4), 2-(4'-Chlorphenyl)-chromanon-(4),
2-(4'-Tolyl)-chromanon-(4),
2-(β-DImethylaminoaethyl)-chromanon-(4),
2-(β-Diaethylaminoaethyl)-chromanon-(4),
2-(γ-Dimethylaminopropyl)-chromanon-(4),
2-(γ-Diaethylaminopropyl)-chromanon-(4),
2-(β-N-Pyrrolidinoaethyl)-chromanon-(4),
2-(β-N-Morpholinoaethyl)-chromanon-(4),
2-(β-N-Piperidinoaethyl)-chromanon-(4),
2-(γ-N-Pyrrolidinopropyl)-chromanon-(4),
2-(γ-N-Morpholinopropyl)-chromanon-(4),
2-(γ-N-Piperidinopropyl)-chromanon-(4),
2-Methyl-2-(γ-diaethylaminopropyl)-chromanon-(4),
2-Methyl-2-(γ-N-pyrrolidinopropyl)-chromanon-(4),
2-Methyl-2-(γ-N-Morpholinopropyl)-chromanon-(4),
2-Methyl-2-(γ-N-Piperidinopropyl)-chromanon-(4),
2-(4'-Oxacyclohexyl)-chromanon-(4),
2-(4'-Methylcyclohexyl)-chromanon-(4),
2-(2'-Methylcyclohexyl)-chromanon-(4),
2-(4'-Methyl-4'-azacyclohexyl)-chromanon-(4),
2-(4'-Benzyl-4'-azacyclohexyl)-chromanon-(4),
2-(4'-Phenyl-4'-azacyclohexyl)-chromanon-(4),
2-(4'-Oxaspirocyclohexa)-chromanon-(4),
2-(4'-Methyl-4'-azaspirocyclohexa)-chromanon-(4),
2-(4'-Benzyl-4'-azaspirocyclohexa)-chromanon-(4),
2-(4'-Phenyl-4'-azaspirocyclohexa)-chromanon-(4),
2-(Cyclohexen-(3)-yl)-chromanon-(4),
6-Chlor-2-spirocyclopenta-chromanon-(4),
6-Chlor-2-spirocyclopenta-chromanon-(4),
6,8-Dichlor-2-spirocyclopenta-chromanon-(4),
6-Methyl-2-spirocyclopentachromanon-(4),
6-isopropyl-2-spirocyclopenta-chromanon-(4),
7-Methyl-2-spirocyclopenta-chromanon-(4),
7-Hydroxy-2-spirocyclopenta-chromanon-(4),

7-Methoxy-2-spirocyclopenta-chromanon-(4),
7-Isopropoxy-2-spirocyclopenta-chromanon-(4),
7-Benzyloxy-2-spirocyclopenta-chromanon-(4),
7-Phenyloxy-2-spirocyclopenta-chromanon-(4),
6,7-Dimethyl-2-spirocyclopenta-chromanon-(4),
5-Methoxy-2-spirocyclopenta-chromanon-(4),
5,6-Benzo-2-spirocyclopenta-chromanon-(4),
7,8-Benzo-2-spirocyclopenta-chromanon-(4),
7-Phenyl-2-spirocyclopenta-chromanon-(4),
8-Chlor-6-methyl-spirocyclopenta-chromanon-(4),
5,7-Dihydroxy-2-spirocyclopenta-chromanon-(4),
6-Chlor-2-spirocyclohexa-chromanon-(4),
7-Chlor-2-spirocyclohexa-chromanon-(4),
6,8-Dichlor-2-spirocyclohexa-chromanon-(4),
6-Methyl-2-spirocyclohexa-chromanon-(4),
6-Isopropyl-2-spirocyclohexa-chromanon-(4),
7-Methyl-2-spirocyclohexa-chromanon-(4),
7-Hydroxy-2-spirocyclohexa-chromanon-(4),
7-Methoxy-2-spirocyclohexa-chromanon-(4),
7-Isopropoxy-2-spirocyclohexa-chromanon-(4),
7-Benzyloxy-2-spirocyclohexa-chromanon-(4),
7-Phenyloxy-2-spirocyclohexa-chromanon-(4),
6,7-Dimethyl-2-spirocyclohexa-chromanon-(4),
5-Methoxy-2-spirocyclohexa-chromanon-(4),
5,6-Benzo-2-spirocyclohexa-chromanon-(4),
7,8-Benzo-2-spirocyclohexa-chromanon-(4),
7-Phenyl-2-spirocyclohexa-chromanon-(4),
8-Chlor-6-methyl-2-spirocyclohexa-chromanon-(4),
5,7-Dihydroxy-2-spirocyclohexa-chromanon-(4),
2-Isopropyl-3-phenyl-6-chlorchromanon-(4),
2-Isopropyl-3-phenyl-6-methylchromanon-(4),
2-Isopropyl-3-phenyl-7-methoxychromanon-(4),
2-Isopropyl-3-p-methoxyphenyl-7-hydroxychromanon-(4).

Die für die Herstellung der erfindungsgemäßen Verbindungen I verwendeten Olefine der Formel III sind bekannt; sie werden technisch sehr häufig eingesetzt und sind dementsprechend in der Literatur reich dokumentiert, vgl. z.B. Beilstein 1, 725 ff; 2, 397 ff; 4, 9 f; 1 III 1866 ff usw.

Als Beispiele für die erfindungsgemäß zu verwendenden Olefine seien genannt:

Acrolein, Crotonaldehyd, Metacrolein, Zimtaldehyd,
4-Chlorzimtaldehyd, 4-Methoxyzimtaldehyd, Methylvinylketon,
Aethylvinylketon, Methyl-isopropenylketon, Mesityloxid,
3-Methyl-3-pentenon-(2), 2-Methyl-1-pentenon-(3),
3-Pentenon-(2), 3-Heptenon-(2), Butyl-vinylketon,
n-Amyl-vinylketon, n-Hexyl-vinylketon, n-Propyl-vinylketon,
Phenylvinylketon, 4-Chlorphenyl-vinylketon,
4-Tolyl-vinylketon, 4-Methoxyphenyl-vinylketon,
Benzylidenaceton, 2-Chlor-benzylidenaceton,
4-Chlor-benzylidenaceton, 4-Methoxy-benzylidenaceton,
4-Isopropyl-benzylidenaceton, Aethylidenacetophenon,
Aethyl-styryl-keton, Isopropyl-styrylketon,
Benzylidencyclohexanon, Benzylidencyclopentanon,
4-Methoxybenzylidencyclohexanon, 4-Chlorbenzylidencyclohexanon,
Benzylidenacetonphenon, Benzyliden-(4'-chloracetophenon),
Benzyliden-(4'-Methylacetophenon),
Benzyliden-(4'-methoxyacetophenon),
(4'-Chlorbenzyliden)-acetophenon,
(4-Methylbenzyliden)-acetophenon,
(4-Methoxybenzyliden)-acetophenon, Dibenzylidenaceton,
Cyclopenten-(2)-on-(1), Cyclohexen-(2)-on-(1),
1-Acetylcyclopenten, 1-Acetylcyclohexen,
2-Methylencyclohexanon, 2-Methylcyclohexen-(2)-on-(1),
Cyclopentylidenaceton, Cyclohexylidenaceton,
Isophoron, Carvon, 1-Propionylcyclohexen,
1-Butyrylcyclohexen, 1-Benzoylcyclohexen,

Cyclohexylidencyclohexanon, Acrylnitril, Crotonnitril, Methacrylnitril, Cyclopentylidenacetonitril, Vinyl-β-methoxycarbonyläthylketon, Vinyl-γ-äthoxycarbonylpropylketon, Cyclohexylidenacetonitril, Zimtsäurenitril, p-Methoxyzimtsäurenitril, 1-Cyanocyclohexen, 1-Cyanocyclopenten, Acrylamid, Methacrylamid, Crotonamid, Acrylsäuredimethylamid, Acrylsäureethylamid, Acrylsäureallylamid, Acrylsäure-tert.-butylamid, Acrylsäureanilid, Acrylsäure-(4-aethoxyanilid), Acrylsäuremethylester, Acrylsäureaethylester, Acrylsäurecyclohexylester, Acrylsäurebenzylester, Methacrylsäuremethylester, Crotonsäuremethylester, Acrylsäure, Methacrylsäure, Crotonsäure, Aethylidenmalonsäurediaethylester, Dimethylmethylenmalonsäurediaethylester, Diaethylmethylenmalonsäurediaethylester, Maleinsäurediaethylester, Fumarsäuredimethylester, Fumarsäurediaethylester, Aethylidencyanessigsäureaethylester, Aethylidencyanessigsäureamid, Cyclohexylidencyanessigsäureaethylester, Aethylidenmalodinitril, Cyclohexylidenmalodinitril, Isobutyrilidencyanessigsäureäthylester, Isobutyrilidencyanessigsäureamid, Isobutyrilidenmalodinitril, Cyclopenten-(1)-carbonsäure-(1)-aethylester, Cyclohexen-(1)-carbonsäure-(1)-aethylester, Zimtsäureaethylester, Zimtsäure, Zimtsäureamid, Zimtsäurediaethylamid, Zimtsäureanilid, 4-Methoxyzimtsäureaethylester, 4-Chlorzimtsäureaethylester, Benzylidenmalonsäuredimethylester, Benzylidenmalonsäurediaethylester, Benzylidencyanessigsäureaethylester, Benzylidenmalondinitril, Benzylidencyanessigsäureamid, β-Acetylacrylsäureaethylester, Aethylidenacetessigsäureaethylester, Aethylidenacetessigsäureamid, Aethylidenacetessigsäureanilid, β-Benzoylacrylsäureaethylester, Benzylidenacetessigsäureaethylester, Benzylidenacetessigsäureamid, Benzylidenacetessigsäureanilid Benzylidenacetylaceton, Aethylidenacetylaceton, 1-Nitro-propen-(1), 2-Nitro-propen-(1), 1-Nitro-buten-(1), 2-Nitro-buten-(1), ω-Nitrostyrol, α-Nitrostilben, Methylvinylsulfon, n-Butylvinylsulfon, Isobutylvinylsulfon, Vinyl-4-tolylsulfon, Phenyl-vinylsulfon, Phenylstyrylsulfon, Vinylsulfonsäureamid, Vinylsulfonsäurediaethylamid, Vinylsulfonsäureanilid, Vinylsulfonsäurepyrrolidid, Vinylsulfonsäuremorpholid, Vinylphosphonsäurediaethylester, Vinylsulfonsäure.

Die erfindungsgemäß verwendeten basischen Hilfsstoffe sind bekannt. Als Beispiele seien genannt Alkalihydroxyde wie Natrium- oder Kaliumhydroxyd, Alkalihydride wie Natrium- oder Kaliumhydrid, Alkalialkoholate wie Natriummethylat, Natriumaethylat, Kaliummethylat, Kaliumaethylat, Kalium-tert.-butylat, metallisches Natrium oder Kalium, Alkaliamide wie Natrium- oder Kaliumamid, Ammoniak sowie organische Stickstoffbasen wie Diaethylamin, Diisopropylamin, Pyridin, Pyrrolidin, Piperidin, Triäthylamin, Tributylamin, Benzyltrimethylammoniumhydroxyd, 1,4-Diaza-bicyclo-[2,2,2]-octan, 1,8-Diazabicyclo[5,4,0]-undecen-(7), 1,5-Diazabicyclo[4,3,0]-nonen-(5). Die basischen Hilfsstoffe werden vorzugsweise in katalytischen Mengen eingesetzt.

Die erfindungsgemäße Reaktion kann oft mit Vorteil in Substanz ausgeführt werden. Sind der eine oder andere oder beide Ausgangsstoffe jedoch Festprodukte mit höherem Schmelzpunkt, so ist die Verwendung von Lösungsmitteln vorteilhaft. Als Lösungsmittel kommen basenstabile, für die erfindungsgemäße Umsetzung inerte Lösungsmittel in Frage, vorzugsweise seien genannt: Alkohole

wie Methanol, Athanol, Isopropanol oder tert.-Butanol, Ather wie Diäthyläther, Diisopropyläther, Tetrahydrofuran oder Dioxan, Kohlenwasserstoffe wie Hexan, Cyclohexan, Benzol oder Toluol oder Mischungen solcher Lösungsmittel.

Die Reaktionstemperaturen können in einem größeren Bereich variiert werden. Im allgemeinen arbeitet man zwischen ca. 0 und ca. 200°C, vorzugsweise von ca. 20—ca. 170°C.

Bei der Durchführung des erfindungsgemäßen Verfahrens setzt man vorzugsweise auf ein Mol Chromanon 1 oder 2 Mol Olefin ein. Überschreitung dieser Mengen ist möglich. Die Reaktionsprodukte können durch Destillation, Auskristallisieren, Einengen und Umlösen oder chromatographische Trennung gewonnen werden.

Als neue Wirkstoffe seien im einzelnen genannt:

3-(γ-Oxopropyl)-2-spirocyclopenta-chromanon-(4),
3-(γ-Oxopropyl)-2-spirocyclohexa-chromanon-(4),
3-(γ-Oxobutyl)-2-spirocyclopenta-chromanon-(4),
3-(γ-Oxobutyl)-2-spirocyclohexa-chromanon-(4),
3-(γ-Oxobutyl)-2,2-dimethyl-chromanon-(4),
3-(γ-Oxobutyl)-2-propyl-chromanon-(4),
3-(γ-Oxobutyl)-2-isopropyl-chromanon-(4),
3-(γ-Oxobutyl)-2,2-diaethyl-chromanon-(4),
3-(γ-Oxobutyl)-2-methyl-2-propyl-chromanon-(4),
3-(γ-Oxobutyl)-2-phenyl-chromanon-(4),
3-(γ-Oxobutyl)-2-cyclohexyl-chromanon-(4),
3-(γ-Oxobutyl)-2-cyclohexen-(3)-yl-chromanon-(4),
3-(γ-Oxobutyl)-2-benzyl-chromanon-(4),
3-(γ-Oxobutyl)-2-phenyläthyl-chromanon-(4),
3-(γ-Oxobutyl)-2-β-phenylpropyl-chromanon-(4),
3-(γ-Oxobutyl)-2-(4′-chlorphenyl)-chromanon-(4),
3-(γ-Oxobutyl)-2-(4′-tolyl)-chromanon-(4),
3-(γ-Oxobutyl)-2-(β-dimethylaminoäthyl)-chromanon-(4),
3-(γ-Oxobutyl)-2-(β-diäthylaminoäthyl)-chromanon-(4),
3-(γ-Oxobutyl)-2-(γ-dimethylaminopropyl)-chromanon-(4),
3-(γ-Oxobutyl)-2-(γ-diäthylaminopropyl)-chromanon-(4),
3-(γ-Oxobutyl)-2-(β-N-pyrrolidinoäthyl)-chromanon-(4),
3-(γ-Oxobutyl)-2-(β-N-morpholinoäthyl)-chromanon-(4),
3-(γ-Oxobutyl)-2-(β-N-piperidinoäthyl)-chromanon-(4),
3-(γ-Oxobutyl)-2-(γ-N-pyrrolidinopropyl)-chromanon-(4),
3-(γ-Oxobutyl)-2-(γ-N-piperidinopropyl)-chromanon-(4),
3-(γ-Oxobutyl)-2-(γ-N-morpholinopropyl)-chromanon-(4),
3-(γ-Oxobutyl)-2-chromanon-(4),
3-(γ-Oxobutyl)-2-methyl-chromanon-(4),
3-(γ-Oxobutyl)-2-aethyl-chromanon-(4),
3-(γ-Oxobutyl)-2-methoxycarbonyl-2-methyl-chromanon-(4),
3-(γ-Oxobutyl)-2-methyl-2-(γ-diäthylaminopropyl)-chromanon-(4),
3-(γ-Oxobutyl)-2-methyl-2-(γ-N-pyrrolidinopropyl)-chromanon-(4),
3-(γ-Oxobutyl)-2-methyl-(γ-N-morpholinopropyl)-chromanon-(4),
3-(γ-Oxobutyl)-2-(γ-N-piperidinopropyl)-chromanon-(4),
3-(γ-Oxobutyl)-2-(4′-Oxacyclohexyl)-chromanon-(4),
3-(γ-Oxobutyl)-2-(4′-methylcyclohexyl)-chromanon-(4),
3-(γ-Oxobutyl)-2-(2′-methylcyclohexyl)-chromanon-(4),
3-(γ-Oxobutyl)-2-(4′-methyl-4′-azacyclohexyl)-chromanon-(4),
3-(γ-Oxobutyl)-2-(4′-benzyl-4′-azacyclohexyl)-chromanon-(4),
3-(γ-Oxobutyl)-2-(4′-phenyl-4′-azacyclohexyl)-chromanon-(4),
3-(γ-Oxobutyl)-2-(4′-oxaspirocyclohexa)-chromanon-(4),
3-(γ-Oxobutyl)-2-(4′-methyl-4′-azaspirocyclohexa)-chromanon-(4),
3-(γ-Oxobutyl)-2-(4′-benzyl-4′-azaspirocyclohexa)-chromanon-(4),
3-(γ-Oxobutyl)-2-(4′-phenyl-4′-azaspirocyclohexa)-chromanon-(4),
3-(γ-Oxobutyl)-2-butyl-chromanon-(4),
3-(γ-Oxobutyl)-2-sec.-butyl-chromanon-(4),
3-(γ-Oxobutyl)-2-isobutyl-chromanon-(4),
3-(γ-Oxobutyl)-2-tert.-butyl-chromanon-(4),
3-(γ-Oxobutyl)-2-(cyclohexen-(3)-yl)-chromanon-(4),
3-(γ-Oxobutyl)-6-chlor-2-spirocyclopenta-chromanon-(4),
3-(γ-Oxobutyl)-7-chlor-2-spirocyclopenta-chromanon-(4),
3-(γ-Oxobutyl)-6,8-dichlor-2-spirocyclopenta-chromanon-(4),
3-(γ-Oxobutyl)-6-methyl-2-spirocyclopenta-chromanon-(4),

3-($\gamma$-Oxobutyl)-6-isopropyl-2-spirocyclopenta-chromanon-(4),
3-($\gamma$-Oxobutyl)-7-methyl-2-spirocyclopenta-chromanon-(4),
3-($\gamma$-Oxobutyl)-7-hydroxy-2-spirocyclopenta-chromanon-(4),
3-($\gamma$-Oxobutyl)-7-methoxy-2-spirocyclopenta-chromanon-(4),
3-($\gamma$-Oxobutyl)-7-isopropoxy-2-spirocyclopenta-chromanon-(4),
3-($\gamma$-Oxobutyl)-7-benzyloxy-2-spirocyclopenta-chromanon-(4),
3-($\gamma$-Oxobutyl)-7-phenyloxy-2-spirocyclopenta-chromanon-(4),
3-($\gamma$-Oxobutyl)-6,7-dimethyl-2-spirocyclopenta-chromanon-(4),
3-($\gamma$-Oxobutyl)-5-methoxy-2-spirocyclopenta-chromanon-(4),
3-($\gamma$-Oxobutyl)-5,6-benzo-2-spirocyclopenta-chromanon-(4),
3-($\gamma$-Oxobutyl)-7,8-benzo-2-spirocyclopenta-chromanon-(4),
3-($\gamma$-Oxobutyl)-7-phenyl-2-spirocyclopenta-chromanon-(4),
3-($\gamma$-Oxobutyl)-8-chlor-6-methyl-2-spirocyclopenta-chromanon-(4),
3-($\gamma$-Oxobutyl)-5,7-dihydroxy-2-spirocyclopenta-chromanon-(4),
3-($\gamma$-Oxobutyl)-6-chlor-2-spirocyclohexa-chromanon-(4),
3-($\gamma$-Oxobutyl)-7-chlor-2-spirocyclohexa-chromanon-(4),
3-($\gamma$-Oxobutyl)-6,8-dichlor-2-spirocyclohexa-chromanon-(4),
3-($\gamma$-Oxobutyl)-6-methyl-2-spirocyclohexa-chromanon-(4),
3-($\gamma$-Oxobutyl)-6-isopropyl-2-spirocyclohexa-chromanon-(4),
3-($\gamma$-Oxobutyl)-7-methyl-2-spirocyclohexa-chromanon-(4),
3-($\gamma$-Oxobutyl)-7-hydroxy-2-spirocyclohexa-chromanon-(4),
3-($\gamma$-Oxobutyl)-7-methoxy-2-spirocyclohexa-chromanon-(4),
3-($\gamma$-Oxobutyl)-7-isopropoxy-2-spirocyclohexa-chromanon-(4),
3-($\gamma$-Oxobutyl)-7-benzyloxy-2-spirocyclohexa-chromanon-(4),
3-($\gamma$-Oxobutyl)-7-phenyloxy-2-spirocyclohexa-chromanon-(4),
3-($\gamma$-Oxobutyl)-6,7-dimethyl-2-spirocyclohexa-chromanon-(4),
3-($\gamma$-Oxobutyl)-5-methoxy-2-spirocyclohexa-chromanon-(4),
3-($\gamma$-Oxobutyl)-5,6-benzo-2-spirocyclohexa-chromanon-(4),
3-($\gamma$-Oxobutyl)-7,8-benzo-2-spirocyclohexa-chromanon-(4),
3-($\gamma$-Oxobutyl)-7-phenyl-2-spirocyclohexa-chromanon-(4),
3-($\gamma$-Oxobutyl)-8-chlor-6-methyl-2-spirocyclohexa-chromanon-(4),
3-($\gamma$-Oxobutyl)-5,7-dihydroxy-2-spirocyclohexa-chromanon-(4),
3-($\gamma$-Oxobutyl)-6-chlor-2,2-dimethyl-2-spirocyclohexa-chromanon-(4),
3-($\gamma$-Oxobutyl)-2,2,6-trimethyl-2-spirocyclohexa-chromanon-(4),
3-($\gamma$-Oxobutyl)-7-hydroxy-2-spirocyclohexa-chromanon-(4),
3-($\gamma$-Oxobutyl)-7-methoxy-2-spirocyclohexa-chromanon-(4),
3-($\gamma$-Oxobutyl)-5,6-benzo-2-spirocyclohexa-chromanon-(4),
3-($\gamma$-Oxobutyl)-7,8-benzo-2-spirocyclohexa-chromanon-(4),
3-($\gamma$-Oxo-hexyl)-2-spirocyclopenta-chromanon-(4),
3-($\gamma$-Oxo-hexyl)-2-spirocyclohexa-chromanon-(4),
3-($\gamma$-Oxo-hexyl)-2,2-dimethyl-chromanon-(4),
3-($\gamma$-Oxo-hexyl)-2-phenyl-chromanon-(4),
3-($\gamma$-Oxo-hexyl)-2-cyclohexyl-chromanon-(4),
3-($\gamma$-Oxo-hexyl)-2-[cyclohexen-(3)-yl]-chromanon-(4),
3-($\gamma$-Oxo-hexyl)-2,2-diaethyl-chromanon-(4),
3-($\gamma$-Oxo-hexyl)-2-propyl-chromanon-(4),
3-($\gamma$-Oxo-hexyl)-2-isopropyl-chromanon-(4),
3-($\gamma$-Oxo-hexyl)-2-hexyl-chromanon-(4),
3-($\gamma$-Oxo-nonyl)-2-spirocyclopenta-chromanon-(4),
3-($\gamma$-Oxo-nonyl)-2-spirocyclohexa-chromanon-(4),
3-($\gamma$-Oxo-nonyl)-2,2-dimethyl-chromanon-(4),
3-($\gamma$-Oxo-nonyl)-2-phenyl-chromanon-(4),
3-($\gamma$-Oxo-nonyl)-2-cyclohexyl-chromanon-(4),
3-($\gamma$-Oxo-nonyl)-2-(cyclohexen-(3')-yl)-chromanon-(4),
3-($\gamma$-Oxo-nonyl)-2,2-diaethyl-chromanon-(4),
3-($\gamma$-Oxo-nonyl)-2-propyl-chromanon-(4),
3-($\gamma$-Oxo-nonyl)-2-isopropyl-chromanon-(4),
3-($\gamma$-Oxo-nonyl)-2-hexyl-chromanon-(4),
3-($\gamma$-Oxo-$\gamma$-phenylpropyl)-2-spirocyclopenta-chromanon-(4),
3-($\gamma$-Oxo-$\gamma$-phenylpropyl)-2-spirocyclohexa-chromanon-(4),
3-($\gamma$-Oxo-$\gamma$-phenylpropyl)-2,2-dimethyl-chromanon-(4),
3-($\gamma$-Oxo-$\gamma$-phenylpropyl)-2-phenyl-chromanon-(4),
3-($\gamma$-Oxo-$\gamma$-phenylpropyl)-2-cyclohexyl-chromanon-(4),
3-($\gamma$-Oxo-$\gamma$-phenylpropyl)-2-(cyclohexen-(3)-yl)-chromanon-(4),
3-($\gamma$-Oxo-$\gamma$-phenylpropyl)-2,2-diäthyl-(3)-chromanon-(4),

3-($\gamma$-Oxo-$\gamma$-phenylpropyl)-2-propyl-(3)-chromanon-(4),
3-($\gamma$-Oxo-$\gamma$-phenylpropyl)-2-isopropyl-(3)-chromanon-(4),
3-($\gamma$-Oxo-$\gamma$-phenylpropyl)-2-hexyl-chromanon-(4),
3-($\alpha$-Methyl-$\gamma$-oxobutyl)-2-spirocyclohexa-chromanon-(4),
3-($\alpha$-Methyl-$\gamma$-oxobutyl)-2-spirocyclopenta-chromanon-(4),
3-($\beta$-Methyl-$\gamma$-oxobutyl)-2-spirocyclopenta-chromanon-(4),
3-($\beta$-Methyl-$\gamma$-oxobutyl)-2-spirocyclohexa-chromanon-(4),
3-($\beta$-Methyl-$\gamma$-oxobutyl)-2,2-dimethyl-chromanon-(4),
3-($\beta$-Methyl-$\gamma$-oxobutyl)-2-phenyl-chromanon-(4),
3-($\beta$-Methyl-$\gamma$-oxobutyl)-2-cyclohexyl-chromanon-(4),
3-($\beta$-Methyl-$\gamma$-oxobutyl)-2-isopropyl-chromanon-(4),
3-($\beta$-Methyl-$\gamma$-oxobutyl)-2-hexyl-chromanon-(4),
3-($\alpha$,$\alpha$-Dimethyl-$\gamma$-oxobutyl)-2-spirocyclopenta-chromanon-(4),
3-($\alpha$,$\alpha$-Dimethyl-$\gamma$-oxobutyl)-2-spirocyclohexa-chromanon-(4),
3-($\alpha$-Phenyl-$\gamma$-oxobutyl)-2-spirocyclopenta-chromanon-(4),
3-[$\alpha$-(4-Chlorphenyl)-$\gamma$-oxobutyl]-2-spirocyclopenta-chromanon-(4),
3-[$\alpha$-(4-Chlorphenyl)-$\gamma$-oxobutyl]-2-spirocyclohexa-chromanon-(4),
3-[$\alpha$-(4-Tolyl)-$\gamma$-oxobutyl]-2-spirocyclopenta-chromanon-(4),
3-[$\alpha$-(4-Tolyl)-$\gamma$-oxobutyl]-2-spirocyclopenta-chromanon-(4),
3-($\alpha$,$\gamma$-Diphenyl-$\gamma$-oxobutyl)-2-spirocyclopenta-chromanon-(4),
3-($\alpha$,$\gamma$-Diphenyl-$\gamma$-oxobutyl)-2-spirocyclohexa-chromanon-(4),
3-[$\alpha$-(4-Chlorphenyl)-$\gamma$-oxobutyl]-2-spirocyclopenta-chromanon-(4),
3-[$\alpha$-(4-Chlorphenyl)-$\gamma$-oxobutyl]-2-spirocyclohexa-chromanon-(4),
3-($\alpha$,$\alpha$-Tetramethylen-$\gamma$-oxobutyl)-2-spirocyclopenta-chromanon-(4),
3-($\alpha$,$\alpha$-Tetramethylen-$\gamma$-oxobutyl)-2-spirocyclohexa-chromanon-(4),
3-($\alpha$,$\alpha$-Pentamethylen-$\gamma$-oxobutyl)-2-spirocyclopenta-chromanon-(4),
3-($\alpha$,$\alpha$-Pentamethylen-$\gamma$-oxobutyl)-2-spirocyclohexa-chromanon-(4),
3-($\alpha$,$\beta$-Trimethylen-$\gamma$-oxobutyl)-2-spirocyclopenta-chromanon-(4),
3-($\alpha$,$\beta$-Trimethylen-$\gamma$-oxobutyl)-2-spirocyclohexa-chromanon-(4),
3-($\alpha$,$\beta$-Tetramethylen-$\gamma$-oxobutyl)-2-spirocyclopenta-chromanon-(4),
3-($\alpha$,$\beta$-Tetramethylen-$\gamma$-oxobutyl)-2-spirocyclohexa-chromanon-(4),
3,3-Di-($\beta$-cyanäthyl)-2-spirocyclopenta-chromanon-(4),
3,3-Di-($\beta$-cyanäthyl)-2-spirocyclohexa-chromanon-(4),
3,3-Di-($\beta$-cyanäthyl)-2,2-dimethyl-chromanon-(4),
3,3-Di-($\beta$-cyanäthyl)-2-propyl-chromanon-(4),
3,3-Di-($\beta$-cyanäthyl)-2-isopropyl-chromanon-(4),
3,3-Di-($\beta$-cyanäthyl)-2,2-diaethyl-chromanon-(4),
3,3-Di-($\beta$-cyanäthyl)-2-methyl-2-propyl-chromanon-(4),
3,3-Di-($\beta$-cyanäthyl)-2-phenyl-chromanon-(4),
3,3-Di-($\beta$-cyanäthyl)-2-cyclohexyl-chromanon-(4),
3,3-Di-($\beta$-cyanäthyl)-2-benzyl-chromanon-(4),
3,3-Di-($\beta$-cyanäthyl)-2-phenyläthyl-chromanon-(4),
3,3-Di-($\beta$-cyanäthyl)-2-phenylpropyl-chromanon-(4),
3,3-Di-($\beta$-cyanäthyl)-2-(4'-chlorphenyl)-chromanon-(4),
3,3-Di-($\beta$-cyanäthyl)-2-(4'-tolyl)-chromanon-(4),
3,3-Di-($\beta$-cyanäthyl)-2-($\beta$-dimethylaminoäthyl)-chromanon-(4),
3,3-Di-($\beta$-cyanäthyl)-2-($\beta$-diäthylaminoäthyl)-chromanon-(4),
3,3-Di-($\beta$-cyanäthyl)-2-($\gamma$-dimethylaminopropyl)-chromanon-(4),
3,3-Di-($\beta$-cyanäthyl)-2-($\gamma$-diäthylaminopropyl)-chromanon-(4),
3,3-Di-($\beta$-cyanäthyl)-2-($\beta$-N-pyrrolidinoäthyl)-chromanon-(4),
3,3-Di-($\beta$-cyanäthyl)-2-($\beta$-N-piperidinoaethyl)-chromanon-(4),
3,3-Di-($\beta$-cyanäthyl)-2-($\beta$-N-morpholinoaethyl)-chromanon-(4),
3,3-Di-($\beta$-cyanäthyl)-2-($\gamma$-N-pyrrolidinopropyl)-chromanon-(4),
3,3-Di-($\beta$-cyanäthyl)-2-($\gamma$-N-piperidinopropyl)-chromanon-(4),
3,3-Di-($\beta$-cyanäthyl)-2-($\gamma$-N-morpholinopropyl)-chromanon-(4),
3,3-Di-($\beta$-cyanäthyl)-chromanon-(4),
3,3-Di-($\beta$-cyanäthyl)-2-methyl-chromanon-(4),
3,3-Di-($\beta$-cyanäthyl)-2-aethyl-chromanon-(4),
3,3-Di-($\beta$-cyanäthyl)-2-methoxycarbonyl-2-methyl-chromanon-(4),
3,3-Di-($\beta$-cyanäthyl)-2-methyl-2-($\gamma$-diaethylaminopropyl)-chromanon-(4),
3,3-Di-($\beta$-cyanäthyl)-2-methyl-2-($\gamma$-N-pyrrolidinopropyl)-chromanon-(4),
3,3-Di-($\beta$-cyanäthyl)-2-methyl-2-($\gamma$-N-morpholinopropyl)-chromanon-(4),
3,3-Di-($\beta$-cyanäthyl)-2-methyl-2-($\gamma$-N-piperidinopropyl)-chromanon-(4),
3,3-Di-($\beta$-cyanäthyl)-2-(4'-oxacyclohexyl)-chromanon-(4),
3,3-Di-($\beta$-cyanaethyl)-2-(4'-methylcyclohexyl)-chromanon-(4),

3,3-Di-(β-cyanaethyl)-2-(2′-methylcyclohexyl)-chromanon-(4),
3,3-Di-(β-cyanaethyl)-2-(4′-methyl-4′-azacyclohexyl)-chromanon-(4),
3,3-Di-(β-cyanaethyl)-2-(4′-benzyl-4′-azacyclohexyl)-chromanon-(4),
3,3-Di-(β-cyanaethyl)-2-(4′-phenyl-4′-azacyclohexyl)-chromanon-(4),
3,3-Di-(β-cyanäthyl)-2-(4′-oxaspirocyclohexa)-chromanon-(4),
3,3-Di-(β-cyanäthyl)-2-(4′-methyl-4′-azaspirocyclohexa)-chromanon-(4),
3,3-Di-(β-cyanäthyl)-2-(4′-benzyl-4′-azaspirocyclohexa)-chromanon-(4),
3,3-Di-(β-cyanäthyl)-2-(4′-phenyl-4′-azaspirocyclohexa)-chromanon-(4),
3,3-Di-(β-cyanäthyl)-2-butyl-chromanon-(4),
3,3-Di-(β-cyanäthyl)-2-sec.-butyl-chromanon-(4),
3,3-Di-(β-cyanäthyl)-2-isobutyl-chromanon-(4),
3,3-Di-(β-cyanäthyl)-2-tert.-butyl-chromanon-(4),
3,3-Di-(β-cyanäthyl)-2-[cyclohexen-(3)-yl]-chromanon-(4),
3,3-Di-(β-cyanäthyl)-6-chlor-2-spirocyclopenta-chromanon-(4),
3,3-Di-(β-cyanäthyl)-7-chlor-2-spirocyclopenta-chromanon-(4),
3,3-Di-(β-cyanäthyl)-6,8-dichlor-2-spirocyclopenta-chromanon-(4),
3,3-Di-(β-cyanäthyl)-6-methyl-2-spirocyclopenta-chromanon-(4),
3,3-Di-(β-cyanäthyl)-6-isopropyl-2-spirocyclopenta-chromanon-(4),
3,3-Di-(β-cyanäthyl)-7-methyl-2-spirocyclopenta-chromanon-(4),
3,3-Di-(β-cyanäthyl)-7-hydroxy-2-spirocyclopenta-chromanon-(4),
3,3-Di-(β-cyanäthyl)-7-methoxy-2-spirocyclopenta-chromanon-(4),
3,3-Di-(β-cyanäthyl)-7-isopropoxy-2-spirocyclopenta-chromanon-(4),
3,3-Di-(β-cyanäthyl)-7-benzyloxy-2-spirocyclopenta-chromanon-(4),
3,3-Di-(β-cyanäthyl)-7-phenyloxy-2-spirocyclopenta-chromanon-(4),
3,3-Di-(β-cyanäthyl)-6,7-dimethyl-2-spirocyclopenta-chromanon-(4),
3,3-Di-(β-cyanäthyl)-5-methoxy-2-spirocyclopenta-chromanon-(4),
3,3-Di-(β-cyanäthyl)-5,6-benzo-2-spirocyclopenta-chromanon-(4),
3,3-Di-(β-cyanäthyl)-7,8-benzo-2-spirocyclopenta-chromanon-(4),
3,3-Di-(β-cyanäthyl)-7-phenyl-2-spirocyclopenta-chromanon-(4),
3,3-Di-(β-cyanäthyl)-8-chlor-6-methyl-2-spirocyclopenta-chromanon-(4),
3,3-Di-(β-cyanäthyl)-5,7-dihydroxy-2-spirocyclopenta-chromanon-(4),
3,3-Di-(β-cyanaethyl)-6-chlor-2-spirocyclohexa-chromanon-(4),
3,3-Di-(β-cyanaethyl)-7-chlor-2-spirocyclohexa-chromanon-(4),
3,3-Di-(β-cyanaethyl)-6,8-dichlor-2-spirocyclohexa-chromanon-(4),
3,3-Di-(β-cyanaethyl)-6-methyl-2-spirocyclohexa-chromanon-(4),
3,3-Di-(β-cyanaethyl)-6-isopropyl-2-spirocyclohexa-chromanon-(4),
3,3-Di-(β-cyanaethyl)-7-methyl-2-spirocyclohexa-chromanon-(4),
3,3-Di-(β-cyanaethyl)-7-hydroxy-2-spirocyclohexa-chromanon-(4),
3,3-Di-(β-cyanaethyl)-7-methoxy-2-spirocyclohexa-chromanon-(4),
3,3-Di-(β-cyanaethyl)-7-isopropoxy-2-spirocyclohexa-chromanon-(4),
3,3-Di-(β-cyanaethyl)-7-benzyloxy-2-spirocyclohexa-chromanon-(4),
3,3-Di-(β-cyanaethyl)-7-phenyloxy-2-spirocyclohexa-chromanon-(4),
3,3-Di-(β-cyanaethyl)-6,7-dimethyl-2-spirocyclohexa-chromanon-(4),
3,3-Di-(β-cyanaethyl)-5-methoxy-2-spirocyclohexa-chromanon-(4),
3,3-Di-(β-cyanaethyl)-5,6-benzo-2-spirocyclohexa-chromanon-(4),
3,3-Di-(β-cyhanaethyl)-7,8-benzo-2-spirocyclohexa-chromanon-(4),
3,3-Di-(β-cyanaethyl)-7-phenyl-2-spirocyclohexa-chromanon-(4),
3,3-Di-(β-cyanaethyl)-8-chlor-6-methyl-2-spirocyclohexa-chromanon-(4),
3,3-Di-(β-cyanaethyl)-5,7-dihydroxy-2-spirocyclohexa-chromanon-(4),
3,3-Di-(β-cyanaethyl)-6-chlor-2,2-dimethyl-2-spirocyclohexa-chromanon-(4),
3,3-Di-(β-cyanaethyl)-6-methyl-2,2-2-spirocyclohexa-chromanon-(4),
3,3-Di-(β-cyanaethyl)-7-hydroxy-2-spirocyclohexa-chromanon-(4),
3,3-Di-(β-cyanaethyl)-7-methoxy-2-spirocyclohexa-chromanon-(4),
3,3-Di-(β-cyanaethyl)-5,6-benzo-2-spirocyclohexa-chromanon-(4),
3,3-Di-(β-cyanaethyl)-7,8-benzo-2-spirocyclohexa-chromanon-(4),
3-(β-Cyanaethyl)-2-spirocyclopenta-chromanon-(4),
3-(β-Cyanaethyl)-2-spirocyclohexa-chromanon-(4),
3-(β-Cyanaethyl)-2,2-dimethyl-chromanon-(4),
3-(β-Cyanaethyl)-2-phenyl-chromanon-(4),
3-(β-Cyanaethyl)-2-cyclohexyl-chromanon-(4),
3-(β-Cyanaethyl)-2-[cyclohexen-(3)-yl]-chromanon-(4),
3-(β-Cyanaethyl)-2,2-diaethyl-chromanon-(4),
3-(β-Cyanaethyl)-2-propyl-chromanon-(4),
3-(β-Cyanaethyl)-2-isopropyl-chromanon-(4),
3-(β-Cyanaethyl)-2-hexyl-chromanon-(4),

3-($\beta$-Cyanpropyl)-2-spirocyclopenta-chromanon-(4),
3-($\beta$-Cyanpropyl)-2-spirocyclohexa-chromanon-(4),
3-($\beta$-Cyanpropyl)-2,2-dimethyl-chromanon-(4),
3-($\beta$-Cyanpropyl)-2-phenyl-chromanon-(4),
3-($\beta$-Cyanpropyl)-2-cyclohexyl-chromanon-(4),
3-($\beta$-Cyanpropyl)-2-[cyclohexen-(3)-yl]-chromanon-(4),
3-($\beta$-Cyanpropyl)-2,2-diaethyl-chromanon-(4),
3-($\beta$-Cyanpropyl)-2-propyl-chromanon-(4),
3-($\beta$-Cyanpropyl)-2-isopropyl-chromanon-(4),
3-($\beta$-Cyanpropyl)-2-hexyl-chromanon-(4),
3,3-Di-($\beta$-Cyanpropyl)-2-spirocyclopenta-chromanon-(4),
3,3-Di-($\beta$-Cyanpropyl)-2-spirocyclohexa-chromanon-(4),
3,3-Di-($\beta$-Cyanpropyl)-2,2-dimethyl-chromanon-(4),
3,3-Di-($\beta$-Cyanpropyl)-2-phenyl-chromanon-(4),
3,3-Di-($\beta$-Cyanpropyl)-2-cyclohexyl-chromanon-(4),
3,3-Di-($\beta$-Cyanpropyl)-2,2-diaethyl-chromanon-(4),
3,3-Di-($\beta$-Cyanpropyl)-2-propyl-chromanon-(4),
3,3-Di-($\beta$-Cyanpropyl)-2-isopropyl-chromanon-(4),
3,3-Di-($\beta$-Cyanpropyl)-2-hexyl-chromanon-(4),
3-($\beta$-Cyanisopropyl)-2-spirocyclopenta-chromanon-(4),
3-($\beta$-Cyanisopropyl)-2-spirocyclohexa-chromanon-(4),
3-($\beta$-Cyanisopropyl)-2,2-dimethyl-chromanon-(4),
3-($\beta$-Cyanisopropyl)-2-phenyl-chromanon-(4),
3-($\beta$-Cyanisopropyl)-2-cyclohexyl-chromanon-(4),
3-($\beta$-Cyanisopropyl)-2,2-diaethyl-chromanon-(4),
3-($\beta$-Cyanisopropyl)-2-propyl-chromanon-(4),
3-($\beta$-Cyanisopropyl)-2-isopropyl-chromanon-(4),
3-($\beta$-Cyanisopropyl)-2-hexyl-chromanon-(4),
3-($\beta$-Cyan-$\alpha$-phenylaethyl)-2-spirocyclopenta-chromanon-(4),
3-($\beta$-Cyan-$\alpha$-phenylaethyl)-2-spirocyclohexa-chromanon-(4),
3-($\beta$-Cyan-$\alpha$-phenylaethyl)-2,2-dimethyl-chromanon-(4),
3-($\beta$-Cyan-$\alpha$-phenylaethyl)-2-phenyl-chromanon-(4),
3-($\beta$-Cyan-$\alpha$-phenylaethyl)-2-cyclohexyl-chromanon-(4),
3-($\beta$-Cyan-$\alpha$-phenylaethyl)-2,2-diaethyl-chromanon-(4),
3-($\beta$-Cyan-$\alpha$-phenylaethyl)-2-propyl-chromanon-(4),
3-($\beta$-Cyan-$\alpha$-phenylaethyl)-2-isopropyl-chromanon-(4),
3-($\beta$-Cyan-$\alpha$-phenylaethyl)-2-hexyl-chromanon-(4),
3-($\beta$-Methoxycarbonylaethyl)-2-spirocyclopenta-chromanon-(4),
3-($\beta$-Methoxycarbonylaethyl)-2-spirocyclohexa-chromanon-(4),
3-($\beta$-Methoxycarbonylaethyl)-2-phenyl-chromanon-(4),
3-($\beta$-Methoxycarbonylaethyl)-2-cyclohexyl-chromanon-(4),
3-($\beta$-Methoxycarbonylaethyl)-2-[cyclohexen-(3)-yl]-chromanon-(4),
3-($\beta$-Methoxycarbonylaethyl)-2,2-diaethyl-chromanon-(4),
3-($\beta$-Methoxycarbonylaethyl)-2-propyl-chromanon-(4),
3-($\beta$-Methoxycarbonylaethyl)-2-isopropyl-chromanon-(4),
3-($\beta$-Methoxycarbonylaethyl)-2-hexyl-chromanon-(4),
3-($\beta$-Aethoxycarbonylaethyl)-2-spirocyclohexa-chromanon-(4)
3-($\beta$-Aethoxycarbonylaethyl)-2-phenyl-chromanon-(4),
3-($\beta$-Allyloxycarbonylaethyl)-2-phenyl-chromanon-(4),
3-($\beta$-Benzyloxycarbonylaethyl)-2-isopropyl-chromanon-(4),
3-($\beta$-Methoxycarbonylpropyl)-2-spirocyclopenta-chromanon-(4),
3-($\beta$-Methoxycarbonylisopropyl)-2-cyclohexyl-chromanon-(4),
3,3-Di-($\beta$-methoxycarbonylaethyl)-2-spirocyclopenta-chromanon-(4),
3,3-Di-($\beta$-methoxycarbonylaethyl)-2-spirocyclohexa-chromanon-(4),
3,3-Di-($\beta$-methoxycarbonylaethyl)-2-phenyl-chromanon-(4),
3,3-Di-($\beta$-methoxycarbonylaethyl)-2-cyclohexyl-chromanon-(4),
3,3-Di-($\beta$-methoxycarbonylaethyl)-2-propyl-chromanon-(4),
3,3-Di-($\beta$-methoxycarbonylaethyl)-2-hexyl-chromanon-(4),
3-($\beta$-Aminocarbonyl-aethyl)-2-spirocyclopenta-chromanon-(4),
3-($\beta$-Aminocarbonyl-aethyl)-2-spirocyclohexa-chromanon-(4),
3-($\beta$-Aminocarbonyl-aethyl)-2,2-dimethyl-chromanon-(4),
3-($\beta$-Aminocarbonyl-aethyl)-2-phenyl-chromanon-(4),
3-($\beta$-Aminocarbonyl-aethyl)-2-cyclohexyl-chromanon-(4),
3-($\beta$-Aminocarbonyl-aethyl)-2-[cyclohexen-(3)-yl]-chromanon-(4),
3-($\beta$-Aminocarbonyl-aethyl)-2,2-diäthyl-chromanon-(4),

3-($\beta$-Aminocarbonyl-aethyl)-2-propyl-chromanon-(4),
3-($\beta$-Aminocarbonyl-aethyl)-2-isopropyl-chromanon-(4),
3-($\beta$-Aminocarbonyl-aethyl)-2-hexyl-chromanon-(4),
3-($\beta$-Aminocarbonylpropyl)-2-spirocyclopenta-chromanon-(4),
3-($\beta$-Aminocarbonylpropyl)-2-phenyl-chromanon-(4),
3-($\beta$-Aminocarbonylpropyl)-2-isopropyl-chromanon-(4),
3-($\beta$-Aminocarbonylisopropyl)-2-spirocyclohexa-chromanon-(4),
3-($\beta$-Aminocarbonylisopropyl)-2-cyclohexyl-chromanon-(4),
3-($\beta$-Aminocarbonylisopropyl)-2-propyl-chromanon-(4),
3-($\beta$-Dimethylaminocarbonylaethyl)-2-spirocyclopenta-chromanon-(4),
3-($\beta$-Dimethylaminocarbonylaethyl)-2-phenyl-chromanon-(4),
3-($\beta$-Allylaminocarbonylaethyl)-2-propyl-chromanon-(4),
3-($\beta$-Allylaminocarbonylaethyl)-2-cyclohexyl-chromanon-(4),
3-($\beta$-Pyrrolidinocarbonylaethyl)-2-spirocyclohexa-chromanon-(4),
3-($\beta$-Pyrrolidinocarbonylaethyl)-2,2-dimethyl-chromanon-(4),
3-(tert.-Butylaminocarbonylaethyl)-2,2-diaethyl-chromanon-(4),
3-(tert.-Butylaminocarbonylaethyl)-2-spirocyclopenta-chromanon-(4),
3-($\beta$-Piperidinocarbonylaethyl)-2-methyl-chromanon-(4),
3-($\beta$-Phenylaminocarbonyläthyl)-2-spirocyclopenta-chromanon-(4), .
3-($\beta$-N-Methylphenylaminocarbonylaethyl)-2-spirocyclopenta-chromanon-(4),
3,3-Di-($\beta$-aminocarbonylaethyl)-2-spirocyclopenta-chromanon-(4),
3,3-Di-($\beta$-aminocarbonylaethyl)-2-spirocyclohexa-chromanon-(4),
3,3-Di-($\beta$-aminocarbonylaethyl)-2-phenyl-chromanon-(4),
3,3-Di-($\beta$-aminocarbonylaethyl)-2-benzyl-chromanon-(4),
3,3-Di-($\beta$-aminocarbonylaethyl)-2,2-dimethyl-chromanon-(4),
3-($\beta$-Aminocarbonylpropyl)-2-spirocyclopenta-chromanon-(4),
3-($\beta$-Aminocarbonylpropyl)-2-phenyl-chromanon-(4),
3-($\beta$-Aminocarbonyl-isopropyl)-2-spirocyclohexa-chromanon-(4)
3-($\beta$-Aminocarbonyl-isopropyl)-2-cyclohexyl-chromanon-(4),
3-($\beta$-Aminocarbonyl-$\alpha$-phenylaethyl)-2,2-dimethyl-chromanon-(4),
3-($\beta$-Aminocarbonyl-$\alpha$-phenylaethyl)-2,2-diaethyl-chromanon-(4),
3-($\beta$-Nitro-$\alpha$-phenylaethyl)-2-spirocyclopenta-chromanon-(4),
3-($\beta$-Nitro-$\alpha$-phenylaethyl)-2-spirocyclohexa-chromanon-(4),
3-($\beta$-Nitro-$\alpha$-phenylaethyl)-2,2-dimethyl-chromanon-(4),
3-($\beta$-Nitro-$\alpha$-phenylaethyl)-2-phenyl-chromanon-(4),
3-($\beta$-Nitro-$\alpha$-phenylaethyl)-2-cyclohexyl-chromanon-(4),
3-($\beta$-Nitro-$\alpha$-phenylaethyl)-2-[cyclohexen-(3)-yl]-chromanon-(4),
3-($\beta$-Nitro-$\alpha$-phenylaethyl)-2,2-diaethyl-chromanon-(4),
3-($\beta$-Nitro-$\alpha$-phenylaethyl)-2-propyl-chromanon-(4),
3-($\beta$-Nitro-$\alpha$-phenylaethyl)-2-isopropyl-chromanon-(4),
3-($\beta$-Nitro-$\alpha$-phenylaethyl)-2-hexyl-chromanon-(4),
3-($\beta$-Nitroisopropyl)-2-spirocyclopenta-chromanon-(4),
3-($\beta$-Nitroisopropyl)-2-phenyl-chromanon-(4),
3-($\beta$-Diaethoxyphosphonylaethyl)-2-spirocyclopenta-chromanon-(4),
3-($\beta$-Diaethoxyphosphonylaethyl)-2-spirocyclohexa-chromanon-(4),
3-($\beta$-Diaethoxyphosphonylaethyl)-2,2-dimethyl-chromanon-(4),
3-($\beta$-Diaethoxyphosphonylaethyl)-2-phenyl-chromanon-(4),
3-($\beta$-Diaethoxyphosphonylaethyl)-2-cyclohexyl-chromanon-(4),
3-($\beta$-Diaethoxyphosphonylaethyl)-2-isopropyl-chromanon-(4),
3-($\beta$-Diaethoxyphosphonylaethyl)-2-hexyl-chromanon-(4),
3-Di-($\beta$-methylsulfonyläthyl)-2-spirocyclopenta-chromanon-(4),
3-Di-($\beta$-methylsulfonyläthyl)-2-spirocyclohexa-chromanon-(4),
3-Di-($\beta$-methylsulfonyläthyl)-2,2-dimethyl-chromanon-(4),
3-Di-($\beta$-methylsulfonyläthyl)-2-propyl-chromanon-(4),
3-Di-($\beta$-methylsulfonyläthyl)-2-isopropyl-chromanon-(4),
3-Di-($\beta$-methylsulfonyläthyl)-2,2-diaethyl-chromanon-(4),
3-Di-($\beta$-methylsulfonyläthyl)-2-methyl-2-propyl-chromanon-(4),
3-Di-($\beta$-methylsulfonyläthyl)-2-phenyl-chromanon-(4),
3-Di-($\beta$-methylsulfonyläthyl)-2-cyclohexyl-chromanon-(4),
3-Di-($\beta$-methylsulfonyläthyl)-2-benzyl-chromanon-(4),
3-Di-($\beta$-methylsulfonyläthyl)-2-phenylaethyl-chromanon-(4),
3-Di-($\beta$-methylsulfonyläthyl)-2-phenylpropyl-chromanon-(4),
3-Di-($\beta$-methylsulfonyläthyl)-2-(4'-chlorphenyl)-chromanon-(4),
3-Di-($\beta$-methylsulfonyläthyl)-2-(4'-tolyl)-chromanon-(4),
3-Di-($\beta$-methylsulfonyläthyl)-2-($\beta$-dimethylaminoaethyl)-chromanon-(4),

3-Di-(β-methylsulfonyläthyl)-2-(β-diaethylaminoaethyl)-chromanon-(4),
3-Di-(β-methylsulfonyläthyl)-2-(γ-dimethylaminopropyl)-chromanon-(4),
3-Di-(β-methylsulfonyläthyl)-2-(γ-diaethylaminopropyl)-chromanon-(4),
3-Di-(β-methylsulfonyläthyl)-2-(β-N-pyrrolidinoaethyl)-chromanon-(4),
3-Di-(β-methylsulfonyläthyl)-2-(β-N-piperidinoaethyl)-chromanon-(4),
3-Di-(β-methylsulfonyläthyl)-2-(β-N-morpholinoaethyl)-chromanon-(4),
3-Di-(β-methylsulfonyläthyl)-2-methyl-2-(γ-diäthylaminopropyl)-chromanon-(4),
3-Di-(β-methylsulfonyläthyl)-2-methyl-2-(γ-N-pyrrolidinopropyl)-chromanon-(4),
3-Di-(β-methylsulfonyläthyl)-2-methyl-2-(γ-N-piperidinopropyl)-chromanon-(4),
3-Di-(β-methylsulfonyläthyl)-2-methyl-2-(γ-N-morpholinopropyl)-chromanon-(4),
3-Di-(β-methylsulfonyläthyl)-chromanon-(4),
3-Di-(β-methylsulfonyläthyl)-2-methyl-chromanon-(4),
3-Di-(β-methylsulfonyläthyl)-2-aethyl-chromanon-(4),
3-Di-(β-methylsulfonyläthyl)-2-methoxycarbonyl-2-methyl-chromanon-(4),
3-Di-(β-methylsulfonyläthyl)-2-(4'-oxacyclohexyl)-chromanon-(4),
3-Di-(β-methylsulfonyläthyl)-2-(4'-methylcyclohexyl)-chromanon-(4),
3-Di-(β-methylsulfonyläthyl)-2-(2'-methyl-cyclohexyl)-chromanon-(4),
3-Di-(β-methylsulfonyläthyl)-2-(4'-methyl-4'-azacyclohexyl)-chromanon-(4),
3-Di-(β-methylsulfonyläthyl)-2-(4'-benzyl-4'-azacyclohexyl)-chromanon-(4),
3-Di-(β-methylsulfonyläthyl)-2-(4'-phenyl-4'-azacyclohexyl)-chromanon-(4),
3-Di-(β-methylsulfonyläthyl)-2-(4'-oxaspirocyclohexa)-chromanon-(4),
3-Di-(β-methylsulfonyläthyl)-2-(4'-methylspirocyclohexa)-chromanon-(4),
3-Di-(β-methylsulfonyläthyl)-2-(4'-methyl-4'-azaspirocyclohexa)-chromanon-(4),
3-Di-(β-methylsulfonyläthyl)-2-(4'-benzyl-4'-azaspirocyclohexa)-chromanon-(4),
3-Di-(β-methylsulfonyläthyl)-2-(4'-phenyl-4'-azaspirocyclohexa)-chromanon-(4),
3-Di-(β-methylsulfonyläthyl)-2-butyl-chromanon-(4),
3-Di-(β-methylsulfonyläthyl)-2-sek.butyl-chromanon-(4),
3-Di-(β-methylsulfonyläthyl)-2-isobutyl-chromanon-(4),
3-Di-(β-methylsulfonyläthyl)-2-tert.-butyl-chromanon-(4),
3-Di-(β-methylsulfonyläthyl)-2-[cyclohexa-(3)-yl]-chromanon-(4),
3-Di-(β-methylsulfonyläthyl)-6-chlor-2-spirocyclopenta-chromanon-(4),
3-Di-(β-methylsulfonyläthyl)-7-chlor-2-spirocyclopenta-chromanon-(4),
3-Di-(β-methylsulfonylmethyl)-6,8-dichlor-2-spirocyclopenta-chromanon-(4),
3-Di-(β-methylsulfonylmethyl)-6-methyl-2-spirocyclopenta-chromanon-(4),
3-Di-(β-methylsulfonylmethyl)-6-isopropyl-2-spirocyclopenta-chromanon-(4),
3-Di-(β-methylsulfonylmethyl)-7-methyl-2-spirocyclopenta-chromanon-(4),
3-Di-(β-methylsulfonylmethyl)-7-hydroxy-2-spirocyclopenta-chromanon-(4),
3-Di-(β-methylsulfonylmethyl)-7-methoxy-2-spirocyclopenta-chromanon-(4),
3-Di-(β-methylsulfonylmethyl)-7-isopropoxy-2-spirocyclopenta-chromanon-(4),
3-Di-(β-methylsulfonylmethyl)-7-benzyloxy-2-spirocyclopenta-chromanon-(4),
3-Di-(β-methylsulfonylmethyl)-7-phenyloxy-2-spirocyclopenta-chromanon-(4),
3-Di-(β-methylsulfonylmethyl)-6,7-dimethyl-2-spirocyclopenta-chromanon-(4),
3-Di-(β-methylsulfonylmethyl)-5-methoxy-2-spirocyclopenta-chromanon-(4),
3-Di-(β-methylsulfonylmethyl)-5,6-benzo-2-spirocyclopenta-chromanon-(4),
3-Di-(β-methylsulfonylmethyl)-7,8-benzo-2-spirocyclopenta-chromanon-(4),
3-Di-(β-methylsulfonylmethyl)-7-phenyl-2-spirocyclopenta-chromanon-(4),
3-Di-(β-methylsulfonylmethyl)-8-chlor-6-methyl-2-spirocyclopenta-chromanon-(4),
3-Di-(β-methylsulfonylmethyl)-5,7-dihydroxy-2-spirocyclopenta-chromanon-(4),
3-Di-(β-methylsulfonylmethyl)-6-chlor-2-spirocyclohexa-chromanon-(4),
3-Di-(β-methylsulfonylmethyl)-7-chlor-2-spirocyclohexa-chromanon-(4),
3-Di-(β-methylsulfonylmethyl)-6,8-dichlor-2-spirocyclohexa-chromanon-(4),
3-Di-(β-methylsulfonylmethyl)-6-methyl-2-spirocyclohexa-chromanon-(4),
3-Di-(β-methylsulfonylmethyl)-6-isopropyl-2-spirocyclohexa-chromanon-(4),
3-Di-(β-methylsulfonylmethyl)-7-methyl-2-spirocyclohexa-chromanon-(4),
3-Di-(β-methylsulfonylmethyl)-7-hydroxy-2-spirocyclohexa-chromanon-(4),
3-Di-(β-methylsulfonyläthyl)-7-methoxy-2-spirocyclohexa-chromanon-(4),
3-Di-(β-methylsulfonyläthyl)-7-isopropoxy-2-spirocyclohexa-chromanon-(4),
3-Di-(β-methylsulfonyläthyl)-7-benzyloxy-2-spirocyclohexa-chromanon-(4),
3-Di-(β-methylsulfonyläthyl)-7-phenyloxy-2-spirocyclohexa-chromanon-(4),
3-Di-(β-methylsulfonyläthyl)-6,7-dimethyl-2-spirocyclohexa-chromanon-(4),
3-Di-(β-methylsulfonyläthyl)-5-methoxy-2-spirocyclohexa-chromanon-(4),
3-Di-(β-methylsulfonyläthyl)-5,6-benzo-2-spirocyclohexa-chromanon-(4),
3-Di-(β-methylsulfonyläthyl)-7,8-benzo-2-spirocyclohexa-chromanon-(4),
3-Di-(β-methylsulfonyläthyl)-7-phenyl-2-spirocyclohexa-chromanon-(4),
3-Di-(β-methylsulfonyläthyl)-8-chlor-6-methyl-2-spirocyclohexa-chromanon-(4),

3-Di-($\beta$-methylsulfonyläthyl)-5,7-dihydroxy-2-spirocyclohexa-chromanon-(4),
3-Di-($\beta$-methylsulfonyläthyl)-6-chlor-2,2-dimethyl-chromanon-(4),
3-Di-($\beta$-methylsulfonyläthyl)-2,2,6-trimethyl-chromanon-(4),
3-Di-($\beta$-methylsulfonyläthyl)-7-hydroxy-2,2-dimethyl-chromanon-(4),
3-Di-($\beta$-methylsulfonyläthyl)-7-methoxy-chromanon-(4),
3-Di-($\beta$-methylsulfonyläthyl)-5,6-benzo-chromanon-(4),
3-Di-($\beta$-methylsulfonyläthyl)-7,8-benzo-chromanon-(4),
3-($\beta$-Phenylsulfonylaethyl)-2-spirocyclopenta-chromanon-(4),
3-($\beta$-Phenylsulfonylaethyl)-2-spirocyclohexa-chromanon-(4),
3-($\beta$-Phenylsulfonylaethyl)-2,2-dimethyl-chromanon-(4),
3-($\beta$-Phenylsulfonylaethyl)-2-propyl-chromanon-(4),
3-($\beta$-Phenylsulfonylaethyl)-2-isopropyl-chromanon-(4),
3-($\beta$-Phenylsulfonylaethyl)-2-cyclohexyl-chromanon-(4),
3-($\beta$-Phenylsulfonylaethyl)-2-phenyl-chromanon-(4),
3-($\beta$-Dimethylaminosulfonylaethyl)-2-spirocyclopenta-chromanon-(4),
3-($\beta$-Dimethylaminosulfonylaethyl)-2-phenyl-chromanon-(4),
3-($\beta$-Pyrrolidinosulfonylaethyl)-2-spirocyclohexa-chromanon-(4),
3-($\beta$-Pyrrolidinosulfonylaethyl)-2-propyl-chromanon-(4),
3-($\beta$-Morpholinosulfonylaethyl)-2-spirocyclopenta-chromanon-(4),
3-($\beta$-Morpholinosulfonylaethyl)-2-hexyl-chromanon-(4),
3-($\alpha$-Phenyl-$\beta$-pyrrolidinosulfonylaethyl)-2-spirocyclopenta-chromanon-(4),
3-($\alpha$-Phenyl-$\beta$-pyrrolidinosulfonylaethyl)-2,2-dimethyl-chromanon-(4),
3-($\alpha$-Phenyl-$\beta$-dimethylaminosulfonylaethyl)-2-spirocyclohexa-chromanon-(4),
3-($\alpha$-Phenyl-$\beta$-dimethylaminosulfonylaethyl)-2-phenyl-chromanon-(4),
3-($\beta$,$\beta$-Dicyanisopropyl)-2-spirocyclopenta-chromanon-(4),
3-($\beta$,$\beta$-Dicyanisopropyl)-2,2-dimethyl-chromanon-(4),
3-($\alpha$-Phenyl-$\beta$-aethoxycarbonyl-$\beta$-cyanäthyl)-2-phenyl-chromanon-(4),
3-($\alpha$-Phenyl-$\beta$-aethoxycarbonyl-$\beta$-cyanäthyl)-2-hexyl-chromanon-(4),
3-($\beta$-Carbonamido-$\beta$-cyanäthyl)-2-spirocyclohexa-chromanon-(4),
3-($\beta$-Carbonamido-$\beta$-cyanäthyl)-2-propyl-chromanon-(4),
3-($\alpha$,$\beta$-Dicyanaethyl)-2,2-diaethyl-chromanon-(4),
3-($\alpha$,$\beta$-Dicyanaethyl)-2-cyclohexyl-chromanon-(4),
3-($\alpha$,$\beta$-Dimethoxycarbonylaethyl)-2-methyl-2-propyl-chromanon-(4),
3-($\alpha$,$\beta$-Dimethoxycarbonylaethyl)-2-isopropyl-chromanon-(4),
3-($\beta$-Acetyl-$\beta$-aethoxycarbonylaethyl)-2-spirocyclopenta-chromanon-(4),
3-($\beta$-Acetyl-$\beta$-aethoxycarbonylaethyl)-2-phenyl-chromanon-(4).

Die erfindungsgemäßen Chromanone der Formel I weisen überraschenderweise die Eigenschaft auf, bei Tieren das Wachstum zu fördern und zu beschleunigen, so daß diese Verbindungen in allen Bereichen der Tierzucht und Tierhaltung zu den genannten Zwecken eingesetzt werden können.

Die Wirksamkeit der erfindungsgemäß verwendeten Verbindungen ist hierbei weitgehend unabhängig von der Art und dem Geschlecht der Tiere. Besonders wertvoll erweisen sich die erfindungsgemäßen Chromanone der Formel I bei der Aufzucht und Haltung von Jung- und Masttieren. Als Tiere, bei denen die Verbindung zu Förderung und Beschleunigung des Wachstums eingesetzt werden kann, seien beispielsweise folgende Nutz- und Ziertiere genannt:

Warmblüter wie Rinder, Schweine, Pferde, Schafe, Ziegen, Katzen, Hunde, Kaninchen, Pelztiere, z.B. Nerze und Chinchilla, Geflügel, z.B. Hühner, Gänse, Enten, Truthähne, Broiler, Tauben, Papageien und Kanarienvögel und Kalblüter, wie Fische, z.B. Karpfen und Reptilien, z.B. Schlangen.

Bevorzugte Anwendung finden Chromanone der Formel I bei der Aufzucht und Haltung von Wiederkäuern wie Kälber, Ziegen, Schafe sowie bei Schweinen und Küken.

Die Menge von Chromanonen der Formel I, die den Tieren zur Erreichung des gewünschten Effektes verabreicht wird, kann weitgehend variiert werden. Sie liegt vorzugsweise bei etwa 0,5 bis 500, insbesondere 1 bis 100 mg/kg Körpergewicht pro Tag. Die Dauer der Verabreichung kann von wenigen Stunden oder Tagen bis zu mehreren Jahren betragen. Die passende Menge Wirkstoff sowie die passende Dauer der Verabreichung hängen insbesondere von der Art, dem Alter, dem Geschlecht, dem Gesundheitszustand und der Art der Haltung der Tiere ab und sind durch jeden Fachmann leicht zu ermitteln.

Die Verbindungen werden den Tieren nach den üblichen Methoden verabreicht. Die Art der Verabreichung hängt insbesondere von der Art, dem Verhalten und dem Gesundheitszustand der Tiere ab. So kann die Verabreichung einmal oder mehrmals täglich in regelmäßigen oder unregelmäßigen Abständen oral oder parenteral erfolgen. Aus Zweckmäßigkeitsgründen ist in dem meisten Fällen eine orale Verabreichung, insbesondere im Rhytmus der Nahrungs- und/oder Getränkeaufnahme der Tiere, vorzuziehen.

Die Verbindungen können als reine Stoffe oder in formulierter Form, also in Mischung mit nichttoxischen inerten Trägerstoffen, als solche sind feste, halbfeste oder flüssige Verdünnungsmittel,

Füllstoffe und Formulierungshilfmittel jeder Art zu verstehen, verabreicht werden.

Chromanone der Formel I können gegebenenfalls in formulierter Form auch zusammen mit pharmazeutischen Wirkstoffen, Mineralsalzen, Spurenelementen, Vitaminen, Eiweißstoffen, Fetten, Farbstoffen und/oder Geschmacksstoffen in geeigneter Form verabreicht werden.

Empfehlenswert ist die orale Verabreichung zusammen mit dem Futter und/oder Trinkwasser, wobei je nach Bedarf die Wirkstoffe der Gesamtmenge oder nur Teilen des Futters und/oder Trinkwasser zugegeben werden.

Die Verbindungen können nach üblichen Methoden durch einfaches Mischen als reine Stoffe, vorzugsweise in fein verteilter Form oder in formulierter Form in Mischung mit eßbaren nichttoxischen Trägerstoffen, gegebenenfalls auch in Form eines Prämix oder eines Futterkonzentrates, dem Futter und/oder Trinkwasser beigefügt werden.

Das Futter und/oder Trinkwasser kann beispielsweise den erfindungsgemäßen Wirkstoff in einer Konzentration von etwa 5 bis 500, insbesondere 5 bis 50 ppm enthalten. Die optimale Höhe der Konzentration des Wirkstoffs in dem Futter und/oder Trinkwasser ist insbesondere abhängig von der Menge der Futter- und/oder Trinkwasseraufnahme der Tiere und kann durch jeden Fachmann leicht ermittelt werden.

Die Art des Futters und seine Zusammensetzung ist hierbei ohne Belang. Es können alle gebräuchlichen, handelsüblichen oder speziellen Futterzusammensetzungen verwendet werden, die vorzugsweise das übliche, für eine ausgewogene Ernährung notwendige Gleichgewicht aus Energie- und Aufbaustoffen einschließlich Vitaminen und Mineralstoffen enthalten. Das Futter kann sich beispeilsweise zusammensetzen aus pflanzlichen Stoffen, z.B. Heu, Rüben, Getreide, Getreidenebenprodukten, tierischen Stoffen, z.B. Fleisch, Fetten, Knochenmehl, Fischprodukten, Vitaminen, z.B. Vitamin A, D-Komplex und B-Komplex, Proteinen, Aminosäuren, z.B. DL-Methionin und anorganischen Stoffen, z.B. Kalk und Kochsalz.

Futterkonzentrate enthalten Chromanone der Formel I neben eßbaren Stoffen, z.B. Roggenmehl, Maismehl, Sojabohnenmehl oder Kalk, gegebenenfalls mit weiteren Nähr- und Aufbaustoffen sowie Proteinen, Mineralsalzen und Vitaminen. Sie können nach den üblichen Mischmethoden hergestellt werden.

Vorzugsweise in Praemixen und Futterkonzentrationen können die Wirkstoffe gegebenenfalls auch durch ein die Oberfläche bedeckendes Mittel, z.B. mit nichttoxischen Wachsen oder Gelatine vor Luft, Licht und/oder Feuchtigkeit geschützt werden.

Beispiel für die Zusammensetzung eines Kükenaufzuchtfutters, das Chromanone der Formel I enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Praemix ergeben nach sorgfältigem Mischen 1 kg Futter.

Die Vitamin-Mineral-Mischung bestehte aus:

6000 I.E. Vitamin A, 1000 I.E. Vitamin $D_3$, 10 mg Vitamin E, 1 mg Vitamin $K_3$, 3 mg Riboflavin, 2 mg Pyridoxin, 20 mg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7H_2O$, 100 mg $FeSO_4 \times 7H_2O$ und 20 mg $CuSO_4 \times 5H_2O$.

Der Wirkstoff-Praemix enthält Chromanone der Formel I in der gewünschten Menge, z.B. 100 mg und zusätzlich 1 g DL-Methionin sowie so viel Sojabohnenmehl, daß 2,5 g Praemix entstehen.

Beispiel für die Zusammensetzung eines Schweineaufzuchtfutters, das den erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmelh, 60 g Sojaschrot, 60 g Tapiokamehl, 38 g Eierhefe, 50 g Vitamin-Mineral-Mischung für Schweine (Zusammensetzung z.B. wie beim Kükenfutter), 30 g Leinkuchenmehl, 30 g Maiskleberfutter, 10 g Sojaöl, 10 g Zuckerrohrmelasse und 2 g Wirkstoff-Praemix (Zusammensetzung z.B. wie beim Kükenfutter) ergeben nach sorgfältigem Mischung 1 kg Futter.

Die angegebenen Futtergemische sind vorzugsweise zur Aufzucht und Mast von Küken bzw. Schweinen abgestimmt, sie können jedoch in gleicher oder ähnlicher Zusammensetzung auch zur Aufzucht und Mast anderer Tiere verwendet werden.

14-tägige Fütterungsversuche an Küken und 6-wöchige Fütterungsversuche an Broilern, die 5 ppm bis 50 ppm Verbindung der Formel I mit dem Futter enthielten, zeigten deutliche Gewichtszunahme bei den mit Chromanonen der Formel I behandelten Tiere im Vergleich zu den ohne Zusatz von Chromanonen der Formel I ernährten Tieren.

Zur Erfindung gehören auch pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen sowie Verfahren zur Herstellung dieser Zubereitungen.

Zur Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2,

1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten genannt.

Tabletten, Dragees, Kapseln, Pillen und Granulate können de oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. GLycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylakohol, Glycerinmonostearat, (h) Adsorptionsmittel, z.B. Kaolin und Bentonit und (i) Gleitmittel, z.B. Talkum-, Calcium- und Magnesiumstearat und feste Polyäthylenglykole oder Gemische der unter (a)—(i) aufgeführten Stoffe.

Die Tabletten, Dragees, Kapseln, Pillen und Granulate können mit den üblichen gegebenenfalls Opakisierungsmittel enthaltenden Ueberzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Aethylalkohol, Isopropylalkohol, Aethylcarbonat, Aethylacetat, Benzylalkohol, Benzylbenzoat, Propylenglykol, 1,3-Butylenglykol, Dimethylformamid, Oele, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Aethylalkohol, Propylenglykol, Suspendiermittel z.B. äthoxylierte Isostearylalkohole, Polyoxyäthylensorbit- und -sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmachsverbessernde Zusätze, z.B. Pfefferminzöl und Eukalyptusöl, und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gewichtsprozent der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur Erfindungn gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung von Cholesterinämien.

Die Wirkstoffe oder die pharmazeutischen Zubereitungen können oral, parenteral, intraperitoneal und/oder rectal, vorzugsweise oral, appliziert werden.

Im allgemeinen hat es sich sowohl in der Human- als auch in der Veterinär-Medizin als vorteilhaft erwiesen, den oder die Wirkstoffe in Mengen von etwa 0,05 bis etwa 500, vorzugsweise 0,5 bis 200 mg/kg Körpergewicht je 24 Stdn., verteilt auf 2 bis 6 Verabreichungen, und zwar vor oder/und während oder/und nach der Mahlzeit zu applizieren. Eine Einzelgabe enthält den oder die Wirkstoffe vorzugsweise in Mengen von etwa 0,1 bis etwa 100 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und

Applikationsart der Wirkstoffe kann durch jeder Fachmann aufgrund seines Fachwissens leicht, erfolgen.

Herstellungsbeispiele

1)

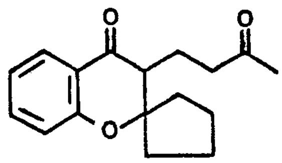

2-Spirocyclopenta-3-(2-γ-oxobutyl)-chromanon-(4)

1616 g (8 m) 2-Spirocyclopenta-chromanon-(4) werden mit einer Mischung von 22 ccm 1,5-Diazabicyclo[4,3,0]-nonen-(5) in 100 ccm abs. Alkohol vorgelegt. Sodann werden unter Rühren und mäßiger Külung mit Eis 1230 g (17,6 m) Methylvinylketon langsam zugetropft. Man hält bei 30°C und rührt anschließend unter Kühlung 6 Stunden nach und läßt über Nacht stehen. Hierauf wird mit dem gleichen Volumen Methylenchlorid verrührt und das Gemisch erst mit 5%iger Essigsäure, dann mit Bicarbonatlösung und schließlich mit Wasser gewaschen. Nach Trocknen mit Natriumsulfat und Einengen wird das Gemisch destilliert und erneut rektifiziert. Man erhält 927 g (42,7% d. Theorie) eines schwach gefärbten Öls vom Kp 158—167°/0,15.

2)

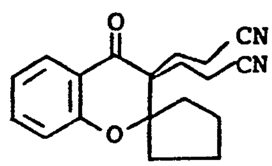

2-Spirocyclopenta-3,3-bis-(2-cyanoäthyl)-chromanon-(4)

202 g (1 m) 2-Spirocyclopenta-chromanon-(4) werden mit 0,8 g Natrium in 15 ml Alkohol vorgelegt. Bei 30—50°C werden 159 g (3 m) frisch destilliertes Acrylnitril zugetropft. Nach Abklingen der leicht exothermen Reaktion wird noch eine Stunde nachgerührt. Nach Abkühlen wird mit wenig Methanol durchgerieben, wobei Kristallisation eintritt. Absaugen und Waschen mit Methanol ergibt 222 g (72% d. Th.). Die Substanz schmilzt nach Umlösen aus Alkohol bei 84—85°C, weiße Kristalle.

3)

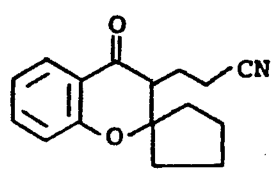

2-Spirocyclopenta-3-(2-cyanäthyl)-chromanon-(4)

40,4 g (0,2 m) 2-Spirocyclopenta-chromanon-(4) werden mit einer Lösung von 0,5 g Natrium in 2,5 ccm Alkohol vorgelegt. Bei 15°C werden unter Kühlen 15,9 g Acrylnitril zugetropft und unter weiterer Kühlung 2 Stunden nachgerührt. Die Verbindung, durch Säulenchromatographie über Kieselgel mit Toluol-Methanol 100:5 in 8 g (15% d. Th.) gewonnen, schmilzt bei 60—62°C., weiße Kristalle.

4)

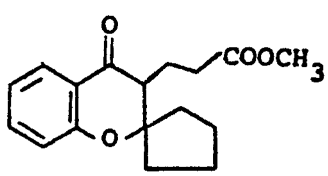

2-Spirocyclopenta-3-(2-carbomethoxyäthyl)-chromanon-(4)

202 g (1 m) 2-Spirocyclopenta-chromanon-(4) werden mit 1,5 g Natriumhydrid versetzt. Bei 30—50°C werden 215 g (2,5 m) Acrylsäuremethylester zugetropft. Anschließend wird 16 Stunden gekocht, nach Abkühlen mit dem gleichen Volumen Methylenchlorid verrührt, mit 5%iger Essigsäure, Bicarbonatlösung und Wasser gewaschen, uber Natriumsulfat getrocknet und eingeengt. Destillation im H.V. ergibt 130 g (45% d. Th.) eines schwach gefärbten Oels vom Kp 156°/0,1.

5) 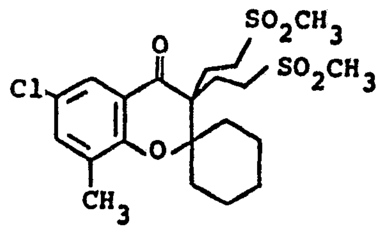

2-Spirocyclohexa-3,3-bis-(2-methylsulfonyläthyl)-6-chlor-8-methyl-chromanon-(4)

49,3 g (0,2 m) 2-Spirocyclohexa-6-chlor-8-methyl-chromanon-(4) und 0,3 g Natrium in 50 ccm Alkohol werden mit 42,4 g (0,4 m) Vinylmethylsulfon versetzt und 2 Stunden zum Sieden erhitzt. Nach dem Abkühlen werden die ausgefallenen Kristalle abgesaugt und mit Aether gewaschen. 55 g weiße Kristalle (60% d. Th.). Nach Umlösen aus Acetonitril Schmp. 209—10°.

Entsprechend dem Verfahren aus den Beispielen 1—5 wurden hergestellt:

6) 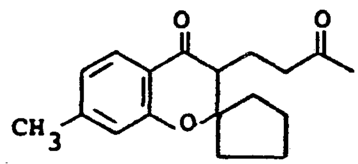 Kp. 155—60°/0,2

7) 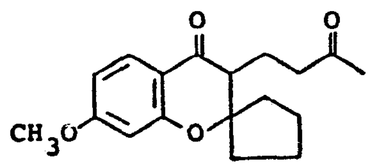 Kp. 160—5°/0,2

8) 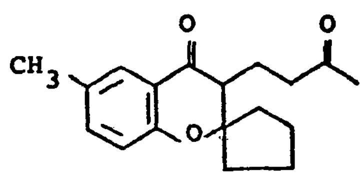 Kp. 155—61/0,2

9) 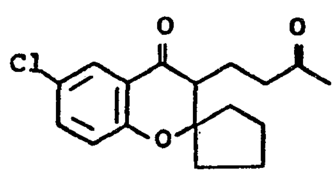 Kp. 170—5°/0,1

10) 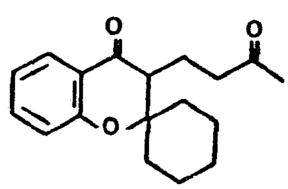 Kp. 160—5°/0,2

11 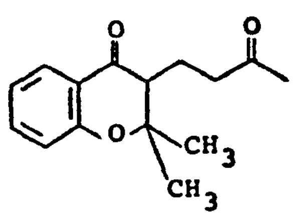 Kp. 120—5/0,2

12) 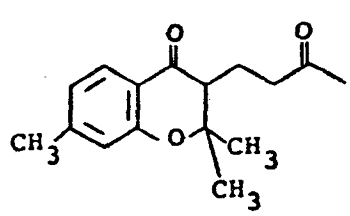 Kp. 135—40°/0,1

13) 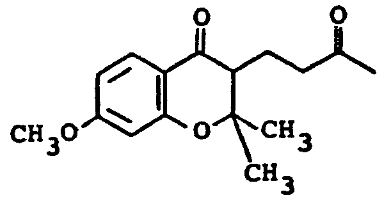 Kp. 155—60°/0,1

14) 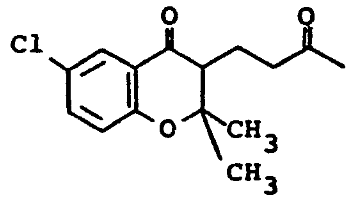 Kp. 135—40°/0,1

15) 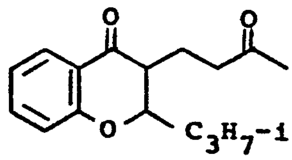 Kp. 155—60°/0,45

16) 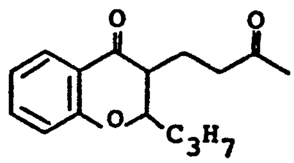 Kp. 165—70/0,5

17) 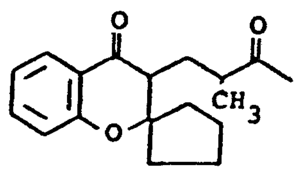 Kp. 155—8°/0,2

18) 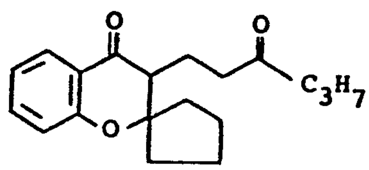 Kp. 165—70/0,2

19) 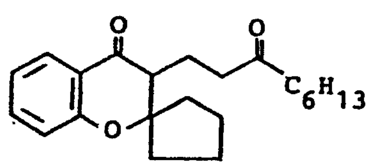 Kp. 185—90°/0,15

20) 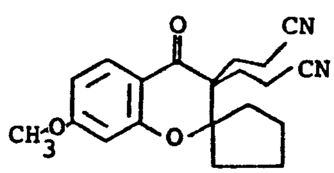 Schmp. 158—61°

21) 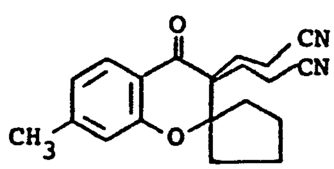 Schmp. 138—40°

22) 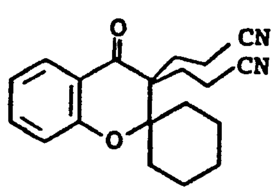 Schmp. 122—3°

23) 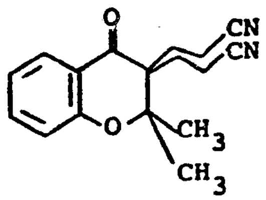 Schmp. 122—3°

24) 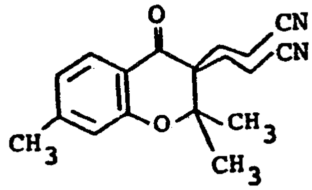 Schmp. 115—6°

25) 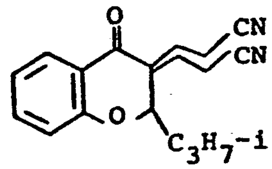 Schmp. 128—9°

26) 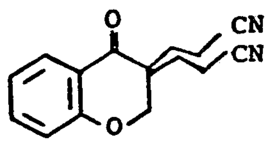 Schmp. 81—3°

27) 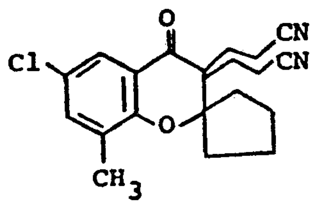 Schmp. 104—6°

28) 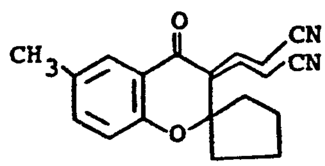 Schmp. 87—8°

29) 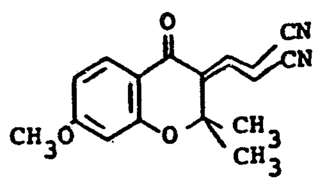 Schmp. 121—2°

30) 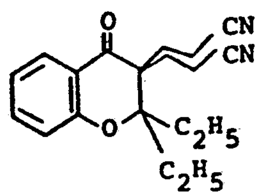 Öl/$n_D^{25}$ = 1.5516

31) 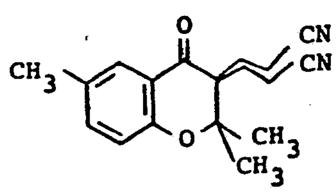 Schmp. 102—4°

32) 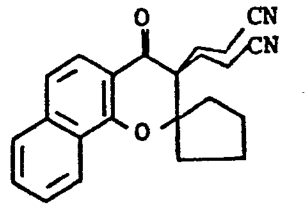 Schmp. 165—7°

33) 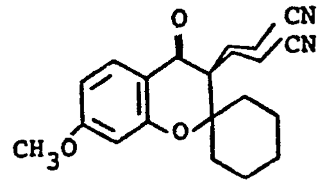 Schmp. 193—6°

34) 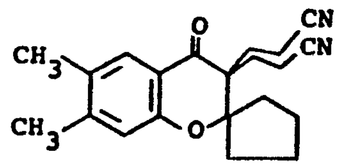 Schmp. 113—5°

35) 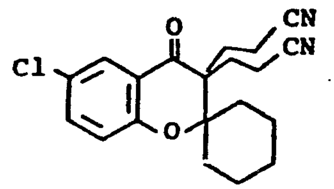 Schmp. 128—30°

36) 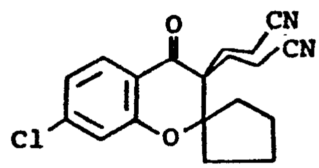 Schmp. 54—5°

37) 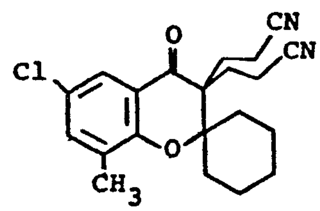 Schmp. 126—7°

38) 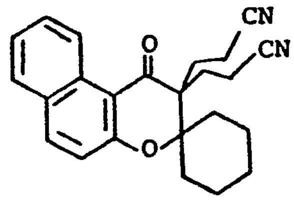 Schmp. 182—4

39) 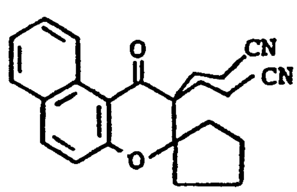 Schmp. 122—4°

40) 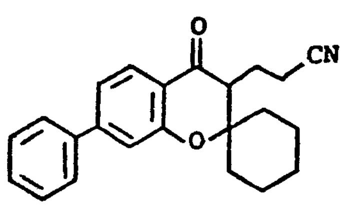 Schmp. 96—7°

41) 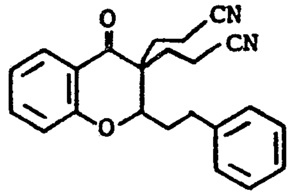 Schmp. 97—100°

42) 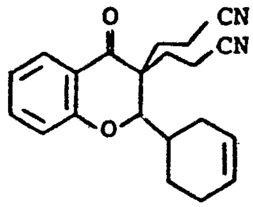 Schmp. 123—5°

43) 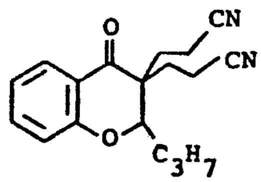 Schmp. 103—4°

44) 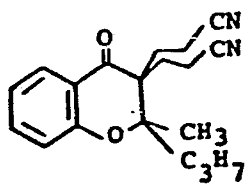 Öl, $n_D^{25} = 1.5482$

45) 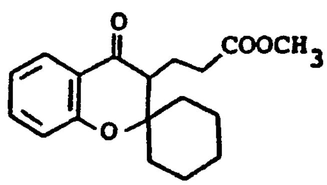 Kp. 200—20°/0,3

46) 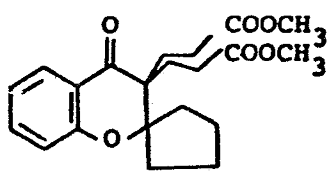 Harz

47) 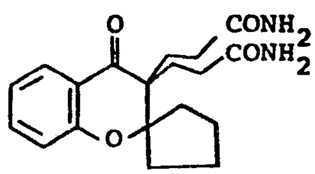 Schmp. 194—6°

48) 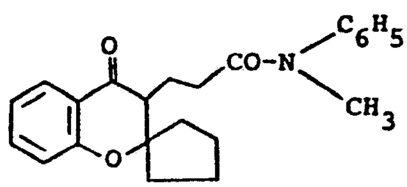 Schmp. 80—2°

49) 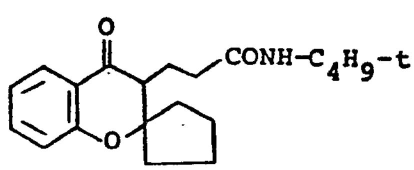 Schmp. 156—8°

50) 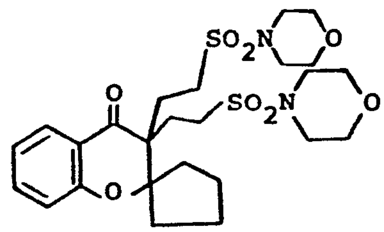 Schmp. 191—2°

51) 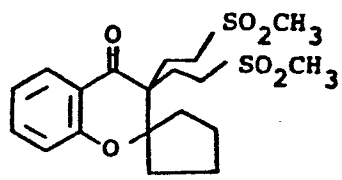 Schmp. 183—4°

52) 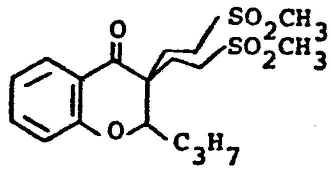 Schmp. 160—1°

53) 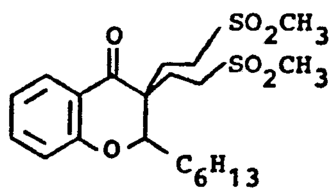 Schmp. 137—8°

54) 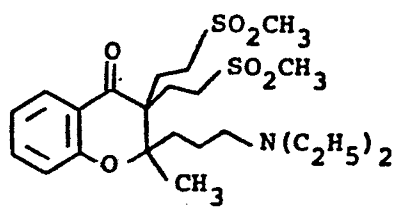 Schmp. 140—2°

55) 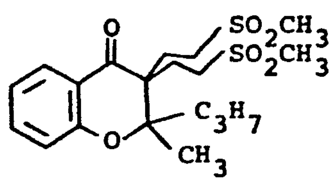 Schmp. 166—8°

56) 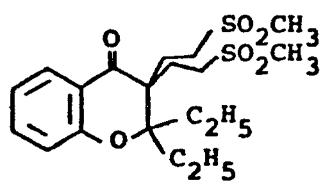 Schmp. 159—62°

57) 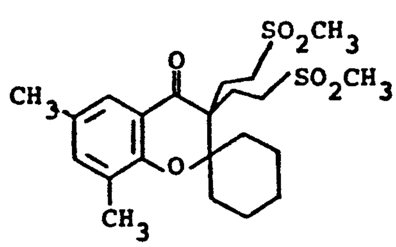 Schmp. 209—10°

58) 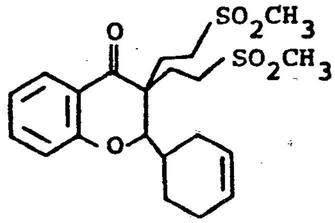 Schmp. 190—1°

59) 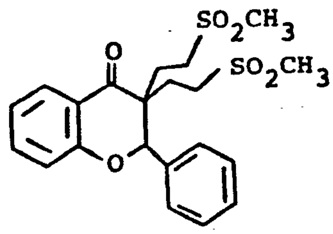 Schmp. 162°

60) 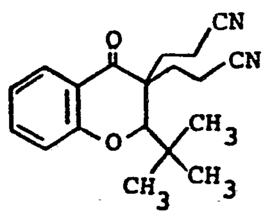 Schmp. 120—2°

61) 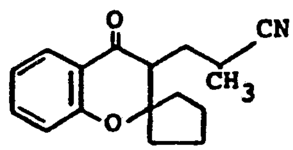 Schmp. 97—9°

62) 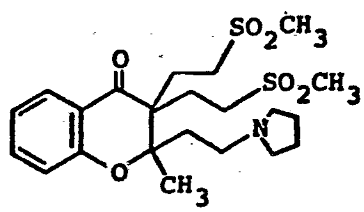 Schmp. 154°

63) 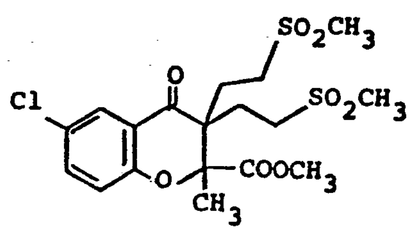 Schmp. 137—8°

64) 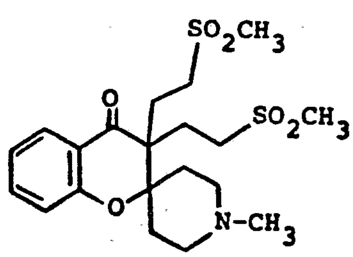 Schmp. 207—9°

65) 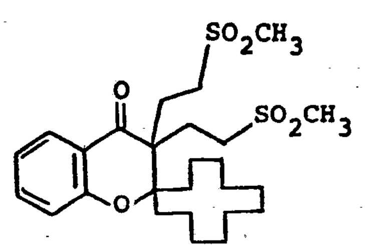 Schmp. 176—8°

66) 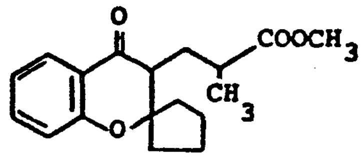 Kp 156—8°/0,15

67) 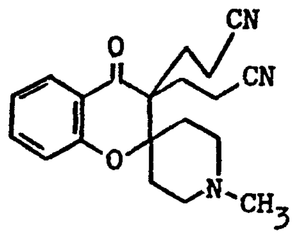 Kp 122—3°

**Patentansprüche**

1. Chromanonderivate der Formel I

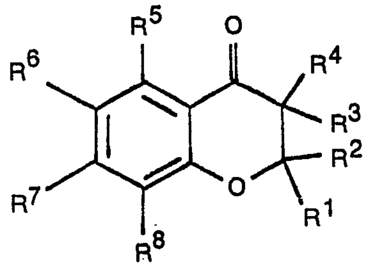 (I)

in welcher

$R^1$ und $R^2$ gleich oder verschieden sind und für Wasserstoff, einen geradkettigen oder verzweigten Alkylrest mit bis zu 18 C-Atomen, einen geradkettigen oder verzweigten Alkylenrest mit bis zu 18 C-Atomen, einen $C_3$—$C_{18}$-Cycloalkylrest, einen $C_3$—$C_{18}$-Cycloalkenylrest, einen $C_6$—$C_{10}$-Arylrest, einen $C_7$—$C_{18}$-Aralkylrest mit 1 bis 8 Kohlenstoffatomen im aliphatischen und 6 bis 10 Kohlenstoffatomen im carbocyclischen Teil, einen $C_2$—$C_7$-Alkoxycarbonylrest, einen Dialkylaminoalkylrest mit 1 bis 6 Kohlenstoffatomen im Alkylteil oder für einen 5 bis 7-gliedrigen Heterocyclenrest mit 1 bis 3 gleichen oder verschiedenen Heteroatomen aus der Reihe Sauerstoff, Schwefel oder Stickstoff stehen oder zusammen mit dem durch sie eingeschlossenen C-Atom einen gesättigten oder ungesättigten carbocyclischen, gegebenenfalls an einen oder mehrere Reste aus der Benzolreihe anellierten, 3- bis 12-gliedrigen Ring bilden oder zusammen mit dem durch sie eingeschlossenen C-Atom einen, gegebenenfalls ein oder mehrere Heteroatome aus der Reihe Sauerstoff, Schwefel oder Stickstoff enthaltenden, 5- bis 12-gliedrigen, gegebenenfalls eine oder zwei Doppelbindungen enthaltenden heterocyclischen Ring bilden,

$R^5$, $R^6$, $R^7$ und $R^8$ gleich oder verschieden sind und für Wasserstoff, Hydroxy, Nitro, Cyano, Carboxy, Halogen, einen geradkettigen oder verzweigten Alkylrest mit bis zu 18 C-Atomen, einen $C_6$—$C_{10}$-Arylrest, einen $C_7$—$C_{18}$-Aralkylrest mit 1 bis 8 Kohlenstoffatomen im aliphatischen und 6 bis 10 Kohlenstoffatomen im carbocyclischen Teil, einen $C_1$—$C_6$-Alkoxyrest, einen $C_2$—$C_7$-Alkoxycarbonylrest, einen $C_6$—$C_{10}$-Aryloxyrest, einen $C_7$—$C_{10}$-Aralkoxyrest, einen Alkylamino-, Alkenylamino-, Arylamino-, Aralkylamino- oder Dialkylaminorest mit bis zu 8 Kohlenstoffatomen oder für Fluor, Chlor, Brom oder Jod stehen,

$R^3$ für den Substituenten

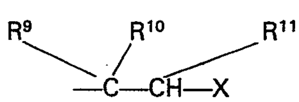

steht, wobei $R^9$, $R^{10}$ und $R^{11}$ gleich oder verschieden sind und für Wasserstoff, Cyano oder Nitro oder für einen geradkettigen oder verzweigten Alkylrest mit bis zu 18 C-Atomen, $C_3$—$C_{18}$-Cycloalkylrest, $C_6$—$C_{10}$-Arylrest, $C_7$—$C_{18}$-Aralkylrest mit 1 bis 8 Kohlenstoffatomen im aliphatischen und 6 bis 10 Kohlenstoffatomen im carbocyclischen Teil, $C_1$—$C_6$-Alkoxyrest, $C_2$—$C_7$-Alkoxycarbonylrest oder $C_1$—$C_6$-Acylrest stehen und X für Cyano, Nitro oder für die Reste $COR^{12}$, $SO_2R^{12}$ oder $PO(R^{12})_2$ steht, in denen $R^{12}$ Wasserstoff, Hydroxy, die Aminogruppe oder die Methylamino-, Äthylamino-, Propylamino-, Isopropylamino-, Dimethylamino-, Diäthylamino, Dipropylamino-, Diisopropylamino-, Phenyläthylamino-, Anilino-, Benzylamino-, Piperidino-, Pyrrolidino-, N-Phenyl-N-methylamino- oder

Morpholinogruppe einen geradkettigen oder verzweigten Alkylrest mit bis zu 18 C-Atomen, $C_6$—$C_{10}$-Arylrest, $C_7$—$C_{18}$-Aralkylrest mit 1 bis 8 Kohlenstoffatomen im aliphatischen Teil und 6 bis 10 Kohlenstoffatomen im carbocyclischen Teil, $C_1$—$C_6$-Alkoxyrest, $C_6$—$C_{10}$-Aryloxyrest, $C_7$—$C_{10}$-Aralkoxyrest, $C_2$—$C_6$-Alkenyloxyrest oder 5 bis 7-gliedrigen Heterocyclenrest mit 1 bis 3 gleichen oder verschiedenen Heteroatomen aus der Reihe Sauerstoff, Schwefel oder Stickstoff bedeutet, oder wobei $R^9$ und $R^{10}$, $R^{10}$ und $R^{11}$, $R^9$ und $R^{12}$ oder $R^{11}$ und $R^{12}$ zusammen mit den durch sie eingeschlossenen Kohlstoffatomen einen gesättigten oder ungesättigten carbocyclischen, gegebenenfalls an einen oder mehrere Reste aus der Benzolreihe anellierten, 3- bis 12-gliedrigen Ring bilden,

$R^4$ für Wasserstoff, $R^1$ oder $R^3$ steht und in welcher die reste $R^1$ bis $R^{12}$ in der Bedeutung Alkyl, Cycloalkyl, Cycloalkenyl, Aryl, Aralkyl, Alkoxy und Alkoxycarbonyl durch Halogen, den Cyanorest, $C_1$—$C_6$-Alkyl, $C_1$—$C_6$-Alkoxy, —$NH_2$, $C_1$—$C_6$-Alkylamino sowie durch Alkoxycarbonyl oder Dialkylamino mit jeweils 1—6 C-Atomen in den Alkyl- bzw. Alkoxyresten substituiert sein können.

2. Chromanonderivate gemäß der Formel I des Anspruchs 1 in welcher

$R^1$ und $R^2$ die in Anspruch 1 angegebene Bedeutungen besitzen,

$R^3$ für den Substituenten

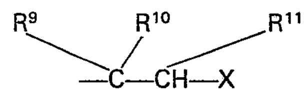

steht, wobei $R^9$, $R^{10}$ und $R^{11}$ gleich oder verschieden sind und für Wasserstoff oder ($C_1$—$C_4$)-Alkyl stehen,

$R^4$ für Wasserstoff, $R^1$ oder $R^3$ steht,

$R^5$, $R^6$, $R^7$ und $R^8$ gleich oder verschieden sind und für Wasserstoff, Halogen, $C_1$—$C_4$-Alkyl, Hydroxy, $C_1$—$C_4$-Alkoxy, $C_6$—$C_{10}$-Aryloxy, $C_7$—$C_{10}$-Aralkoxy steht,

X für die Cyanogruppe oder die Reste $COR^{12}$ oder $SO_2R^{12}$ steht,

wobei $R^{12}$ für $C_1$—$C_{12}$-Alkyl steht.

3. Verfahren zur Herstellung von Chromanonderivaten der Formel I

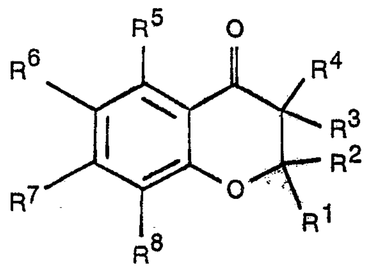

(I)

in welcher

die Substituenten $R^1$ bis $R^{12}$ die in Anspruch 1 angegebene Bedeutung haben, dadurch gekennzeichnet, daß man Chromanone der Formel II

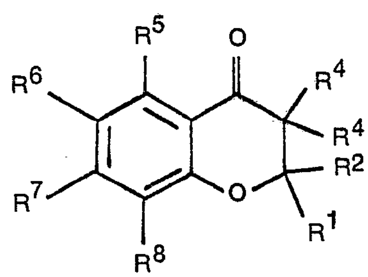

(II)

in welcher

$R^1$, $R^2$, $R^4$, $R^5$, $R^6$, $R^7$ und $R^8$ die in Anspruch 1 angegebene Bedeutung besitzen mit Olefinen der Formel III

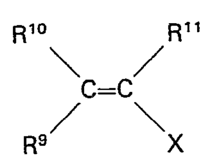

(III)

in welcher

$R^9$, $R^{10}$, $R^{11}$ und X die in Anspruch 1 angegebene Bedeutung haben,

in Gegenwart basischer Hilfsstoffe umsetzt und die Reaktionsprodukte nach an sich bekannten Methoden isoliert.

4. Arzneimittel, gekennzeichnet, durch einen Gehalt an mindestens einem Chromanon-Derivaten gemäß Anspruch 1.

5. Nutritives Mittel für Tiere, gekennzeichnet durch einen Gehalt an mindestens einem Chromanon-Derivat gemäß Anspruch 1.

6. Chromanone der Formel I gemäß Anspruch 1 zur Verwendung bei der Bekämpfung der Cholesterinämie.

7. Chromanone der Formel I gemäß Anspruch 1 zur Förderung und Beschleunigung des Wachstums und zur Verbesserung der Futterverwertung bei Tieren.

**Claims**

1. Chromanone derivatives of the formula I

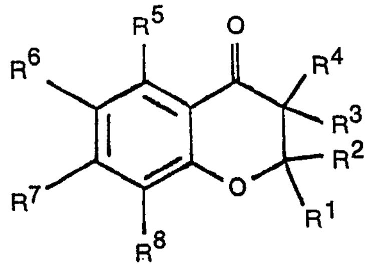

(I)

in which

$R^1$ and $R^2$ are identical or different and represent hydrogen, a straight-chain or branched alkyl radical with up to 18 C-atoms, a straight-chain or branched alkylene radical with up to 18 C-atoms, a $C_3$—$C_{18}$-cycloalkyl radical, a $C_3$—$C_{18}$-cycloalkenyl radical, a $C_6$—$C_{10}$-aryl radical, a $C_7$—$C_{18}$-aralkyl radical with 1 to 8 carbon atoms in the aliphatic part and 6 to 10 carbon atoms in the carbocyclic part, a $C_2$—$C_7$-alkoxycarbonyl radical, a dialkylaminoalkyl radical with 1 to 6 carbon atoms in the alkyl part, or a 5-membered to 7-membered heterocyclic radical with 1 to 3 identical or different hetero-atoms from the group comprising oxygen, sulphur or nitrogen, or together with the C-atom between them form a saturated or unsaturated carbocyclic, 3-membered to 12-membered ring which is optionally fused onto one or more radicals from the benzene series, or together with the C-atom between them form a 5-membered to 12-membered heterocyclic ring which optionally contains one or more hetero-atoms from the group comprising oxygen, sulphur or nitrogen and which optionally contains one or two double bonds,

$R^5$, $R^6$, $R^7$ and $R^8$ are identical or different and represent hydrogen, hydroxy, nitro, cyano, carboxy, halogen, a straight-chain or branched alkyl radical with up to 18 C-atoms, a $C_6$—$C_{10}$-aryl radical, a $C_7$—$C_{18}$-aralkyl radical with 1 to 8 carbon atoms in the aliphatic part and 6 to 10 carbon atoms in the carbocyclic part, a $C_1$—$C_6$-alkoxy radical, a $C_2$—$C_7$-alkoxycarbonyl radical, a $C_6$—$C_{10}$-aryloxy radical, a $C_7$—$C_{10}$-aralkoxy radical, an alkylamino, alkenylamino, arylamino, aralkylamino or dialkylamino radical with up to 8 carbon atoms, or fluorine, chlorine, bromine or iodine,

$R^3$ represents the substituent

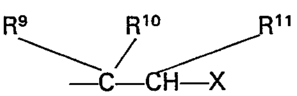

wherein

$R^9$, $R^{10}$ and $R^{11}$ are identical or different and represent hydrogen, cyano or nitro, or a straight-chain or branched alkyl radical with up to 18 C-atoms, a $C_3$—$C_{18}$-cycloalkyl radical, a $C_6$—$C_{10}$-aryl radical, a $C_7$—$C_{18}$-aralkyl radical with 1 to 8 carbon atoms in the aliphatic part and 6 to 10 carbon atoms in the carbocyclic part, a $C_1$—$C_6$-alkoxy radical, a $C_2$—$C_7$-alkoxycarbonyl radical or a $C_1$—$C_6$-acyl radical and

X represents cyano, nitro or the radicals $COR^{12}$, $SO_2R^{12}$ or $PO(R^{12})_2$, in which $R^{12}$ represents hydrogen, hydroxy, the amino group or the methylamino, ethylamino, propylamino, isopropylamino, dimethylamino, diethylamino, dipropylamino, diisopropylamino, phenylethylamino, phenylethylamino, anilino, benzylamino, piperidino, pyrrolidino, N-phenyl-N-methylamino or morpholino group, a straight-chain or branched alkyl radical with up to 18 C-atoms, a $C_6$—$C_{10}$-aryl radical, a $C_7$—$C_{18}$-aralkyl radical with 1 to 8 carbon atoms in the aliphatic part and 6 to 10 carbon atoms in the carbocyclic part, a $C_1$—$C_6$-alkoxy radical, a $C_6$—$C_{10}$-aryloxy radical, a $C_7$—$C_{10}$-aralkoxy radical, a $C_2$—$C_6$-alkenyloxy radical, or a 5-membered to 7-membered heterocyclic radical with 1 to 3 identical or different hetero-atoms from the group comprising oxygen, sulphur or nitrogen,

or wherein

$R^9$ and $R^{10}$, $R^{10}$ and $R^{11}$, $R^9$ and $R^{12}$ or $R^{11}$ and $R^{12}$ together with the carbon atoms between them form a saturated or unsaturated carbocyclic, 3-membered to 12-membered ring which is optionally fused onto one or more radicals from the benzene series,

$R^4$ represents hydrogen, $R^1$ or $R^3$

and in which the radicals $R^1$ to $R^{12}$, when they denote alkyl, cycloalkyl, cycloalkenyl, aryl, aralkyl, alkoxy and alkoxycarbonyl, may be substituted by halogen, the cyano radical, $C_1$—$C_6$-alkyl, $C_1$—$C_6$-alkoxy, —$NH_2$, $C_1$—$C_6$-alkylamino, as well as by alkoxycarbonyl or dialkylamino with in each case 1—6 C-atoms in the alkyl and alkoxy radicals.

2. Chromanone derivatives according to formula I of Claim 1, in which

$R^1$ and $R^2$ have the meanings indicated in Claim 1,

$R^3$ represents the substituent

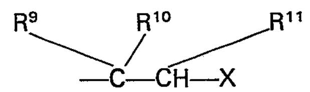

wherein $R^9$, $R^{10}$ and $R^{11}$ are identical or different and represent hydrogen or ($C_1$—$C_4$)-alkyl,

$R^4$ represents hydrogen, $R^1$ or $R^3$,

$R^5$, $R^6$, $R^7$ and $R^8$ are identical or different and represent hydrogen, halogen $C_1$—$C_4$-alkyl, hydroxy, $C_1$—$C_4$-alkoxy, $C_6$—$C_{10}$-aryloxy, $C_7$—$C_{10}$-aralkoxy,

X represents the cyano group or the radicals $COR^{12}$ or $SO_2R^{12}$,

wherein $R^{12}$ represents $C_1$—$C_{12}$-alkyl.

3. Process for the production of chromanone derivatives of the formula I

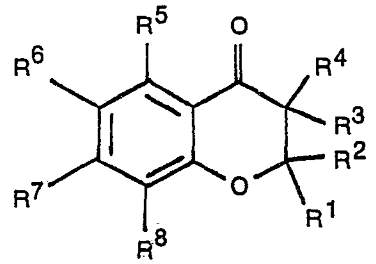

(I)

in which the substituents $R^1$ to $R^{12}$ have the meaning indicated in Claim 1, characterised in that chromanones of the formula II

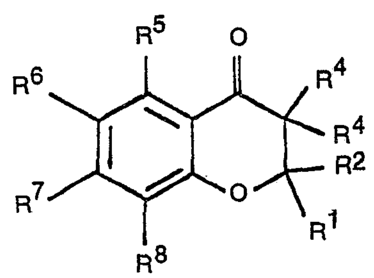

(II)

in which $R^1$, $R^2$, $R^4$, $R^5$, $R^7$ and $R^8$ have the meaning indicated in Claim 1, are reacted with olefines of the formula III

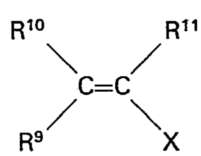

(III)

in which $R^9$, $R^{10}$, $R^{11}$ and X have the meaning indicated in Claim 1, in the presence of basic auxiliaries and the reaction products are isolated according to methods known per se.

4. A medicament, characterised in that it contains at least one chromanone derivative according to Claim 1.

5. A nutritive material for animals, characterised in that it contains at least one chromanone derivative according to Claim 1.

6. Chromanones of the formula I according to Claim 1 for use in combating cholesterolaemia.

7. Chromanones of the formula I according to Claim 1 for promoting and accelerating the growth of animals and for improving the utilization of feed in animals.

**Revendications**

1. Dérivés de chromanone de formule I:

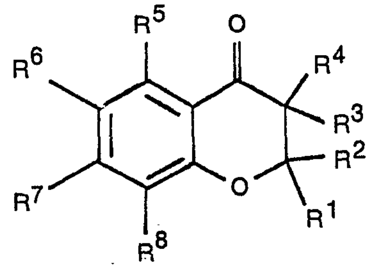

(I)

dans laquelle:

$R^1$ et $R^2$ sont égaux ou différents et représentent de l'hydrogène, un reste alkyle à chaîne droite ou ramifié ayant jusqu'à 18 atomes de carbone, un reste alkylène à chaîne droite ou ramifié ayant jusqu'à 18 atomes de carbone, un reste cycloalkyle en $C_3$ à $C_{18}$, un reste cycloalcényle en $C_3$ à $C_{18}$, un reste aryle et $C_6$ à $C_{10}$, un reste aralkyle en $C_7$ à $C_{18}$ ayant 1 à 8 atomes de carbone dans la partie aliphatique et 6 à 10 atomes de carbone dans la partie carbocyclique, un reste alkoxycarbonyle en $C_2$ à $C_7$, un reste dialkylamino-alkyle ayant 1 à 6 atomes de carbone dans la partie alkyle ou un reste hétérocyclique pentagonal à heptagonal ayant 1 à 3 hétéro-atomes égaux ou différents de la série de l'oxygène, du soufre ou de l'azote, ou forment conjointement avec l'atome de carbone qu'ils encadrent, un noyau carbocyclique saturé ou non saturé de 3 à 12 chaînons, éventuellement condensé sur un ou plusieurs restes de la série benzénique, ou forment conjointement avec l'atome de carbone qu'ils encadrent, un noyau hétérocyclique de 5 à 12 chaînons comprenant éventuellement un ou plusieurs hétéro-atomes de la série de l'oxygène, du soufre ou de l'azote, comportant éventuellement une ou deux doubles liaisons,

$R^5$, $R^6$, $R^7$ et $R^8$ sont égaux ou différents et représentent l'hydrogène, un reste hydroxy, nitro, cyano, carboxy, un halogène, un reste alkyle à chaîne droite ou ramifié ayant jusqu'à 18 atomes de carbone, un reste aryle en $C_6$ à $C_{10}$, un reste aralkyle en $C_7$ à $C_{18}$ ayant 1 à 8 atomes de carbone dans la partie aliphatique et 6 à 10 atomes de carbone dans la partie carbocyclique, un reste alkoxy en $C_1$ à $C_6$, un reste alkoxycarbonyle en $C_2$ à $C_7$, un reste aryloxy en $C_6$ à $C_{10}$, un reste aralkoxy en $C_7$ à $C_{10}$, un reste alkylamino, alcénylamino, arylamino, aralkylamino ou dialkylamino ayant jusqu'à 8 atomes de carbone ou du fluor, du chlore, du brome ou de l'iode,

$R^3$ représente le substituant:

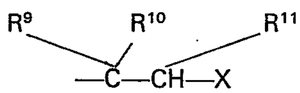

où $R^9$, $R^{10}$ et $R^{11}$ sont égaux ou différents et représentent de l'hydrogène, un reste cyano ou nitro ou un reste alkyle à chaîne droite ou ramifié ayant jusqu'à 18 atomes de carbone, un reste cycloalkyle en $C_3$ à $C_{18}$, un reste aryle en $C_6$ à $C_{10}$, un reste aralkyle en $C_7$ à $C_{18}$ ayant 1 à 8 atomes de carbone dans la partie aliphatique et 6 à 10 atomes de carbone dans la partie carbocyclique, un reste alkoxy en $C_1$ à $C_6$, alkoxycarbonyle en $C_2$ à $C_7$ ou acyle en $C_1$ à $C_6$ et

X est un radical cyano, nitro ou les restes $COR^{12}$, $SO_2R^{12}$ ou $PO(R^{12})_2$ dans lesquels $R^{12}$ est l'hydrogène, un radical hydroxy, le groupe amino ou le groupe méthylamino, éthylamino, propylamino, isopropylamino, diméthylamino, diéthylamino, dipropylamino, diisopropylamino, phényléthylamino, phényléthylamino, anilino, benzylamino, pipéridino, pyrrolidino, N-phényl-N-méthylamino ou morpholino, un reste alkyle à chaîne droite ou ramifié ayant jusqu'à 18 atomes de carbone, un reste aryle en $C_6$ à $C_{10}$, un reste aralkyle en $C_7$ à $C_{18}$ ayant 1 à 8 atomes de carbone dans la partie aliphatique et 6 à 10 atomes de carbone dans la partie carbocyclique, un reste alkoxy en $C_1$ à $C_6$, un reste aryloxy en $C_6$ à $C_{10}$, un reste aralkoxy en $C_7$ à $C_{10}$, un reste alcényloxy en $C_2$ à $C_6$ ou un reste hétérocyclique de 5 à 7 chaînons ayant 1 à 3 hétéro-atomes égaux ou différents de la série de l'oxygène, du soufre ou de l'azote, ou bien $R^9$ et $R^{10}$, $R^{10}$ et $R^{11}$, $R^9$ et $R^{12}$ ou $R^{11}$ et $R^{12}$ forment conjointement avec les atomes de carbone qu'ils encadrent un noyau de 3 à 12 chaînons saturés ou non saturés carbocycliques, éventuellement condensés sur un ou plusieurs restes de la série benzénique,

$R^4$ représente de l'hydrogène, un reste $R^1$ ou $R^3$, et dans laquelle les restes $R^1$ à $R^{12}$, lorsqu'ils signifient un reste alkyle, cycloalkyle, cycloalcényle, aryle, aralkyle, alkoxy et alkoxycarbonyle, peuvent être substitués par un halogène, le reste cyano, un reste alkyle en $C_1$ à $C_6$, alkoxy en $C_1$ à $C_6$, $—NH_2$, alkylamino en $C_1$ à $C_6$ ainsi que par un reste alkoxycarbonyle ou dialkylamino ayant chacun 1 à 6 atomes de carbone dans les restes alkyle et alkoxy.

2. Dérivés de chromanone de formule I suivant la revendication 1, dans laquelle

$R^1$ et $R^2$ ont les définitions indiquées dans la revendication 1,

$R^3$ représente le substituant

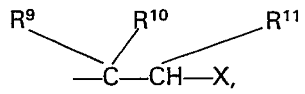

où

$R^9$, $R^{10}$ et $R^{11}$ sont égaux ou différents et représentent l'hydrogène ou un reste alkyle en $C_1$ à $C_4$,

$R^4$ est l'hydrogène, $R^1$ ou $R^3$,

$R^5$, $R^6$, $R^7$ et $R^8$ sont égaux ou différents et représentent l'hydrogène, un halogène, un reste alkyle en $C_1$ à $C_4$, hydroxy, alkoxy en $C_1$ à $C_4$, aryloxy en $C_6$ à $C_{10}$, aralkoxy en $C_7$ à $C_{10}$,

X est le groupe cyano ou les restes $COR^{12}$ ou $SO_2R^{12}$,

$R^{12}$ étant un radical alkyle en $C_1$ à $C_{12}$.

3. Procédé de production de dérivés de chromanone de formule I:

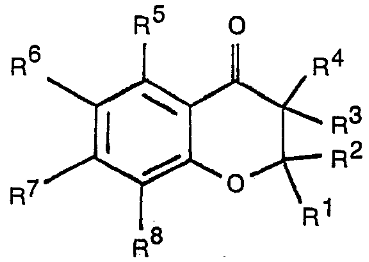

(I)

où

les substituants $R^1$ à $R^{12}$ ont la définition indiquée dans la revendication 1, caractérisé en ce qu'on fait réagir des chromanones de formule II: .

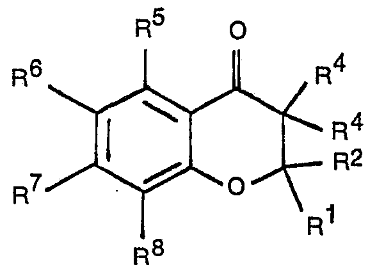

(II)

dans laquelle

$R^1$, $R^2$, $R^4$, $R^5$, $R^7$ et $R^8$ ont la définition indiquée dans la revendication 1, avec des oléfines de formule III:

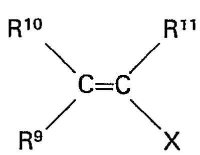

(III)

dans laquelle:

$R^9$, $R^{10}$, $R^{11}$ et X ont la définition indiquée dans la revendication 1, en présence de substances auxiliaires basiques et on isole les produits de réaction par des opérations connues.

4. Médicament, caractérisé par une teneur en ou moins un dérivé de chromanone suivant la revendication 1.

5. Composition nutritive pour animaux, caractérisée par une teneur en au moins un dérivé de chromanone suivant la revendication 1.

6. Chromanones de formule I suivant la revendication 1, destinées à être utilisées pour combattre la cholestérolémie.

7. Chromanones de formule I suivant la revendication 1, destinées à l'activation et à l'accélération de la croissance et à l'amélioration de l'assimilation de la nourriture chez les animaux.